# EUROPEAN PATENT SPECIFICATION

(11) **EP 4 079 744 B1**
(45) Date of publication and mention of the grant of the patent: **06.08.2025**
(21) Application number: 21170258.4
(22) Date of filing: 23.04.2021
(51) Int. Cl.: C07H 17/08, A01N 43/22, A01P 7/00

(54) **NOVEL SPINOSYN COMPOUNDS AND THEIR USE AS A PESTICIDE**
NEUE SPINOSYNVERBINDUNGEN UND IHRE VERWENDUNG ALS PESTIZIDE MITTEL
NOUVEAUX COMPOSÉS DE SPINOSYNE ET LEUR UTILISATION COMME PESTICIDES

(43) Date of publication of application: 26.10.2022
(73) Proprietor: Acies Bio d.o.o., 1000 Ljubljana (SI)
(72) Inventor: Kosec, Gregor, 1000 Ljubljana (SI); Krajnc, Nika Lendero, 3310 Zalec (SI); Fujs, Stefan, 1000 Ljubljana (SI); Camp, Nicholas, London EC4A 3TW (GB)
(74) Representative: Zacco GmbH

(56) References cited:
- WO-A1-2020/023943
- WO-A1-97/00265
- CN-A- 102 190 694
- CN-A- 111 632 061
- US-B1- 6 919 464
- DEAMICIS CARL V ET AL: "Physical and biological properties of the spinosyns: novel macrolide pest-control agents from fermentation", CHEMISTRY, PROCESS DESIGN, AND SAFETY FOR THE NITRATION INDUSTRY /ACS /SYMPOSIUM SERIES, AMERICAN CHEMICAL SOCIETY/OXFORD UNIVERSITY PRESS, US, vol. 658, 1 January 1997 (1997-01-01), pages 144 - 154, XP009125573, ISSN: 0097-6156, DOI: 10.1021/BK-1997-0658.CH011
- HERBERT A KIRST: "The spinosyn family of insecticides: realizing the potential of natural products research", THE JOURNAL OF ANTIBIOTICS, vol. 63, no. 3, 12 February 2010 (2010-02-12), pages 101 - 111, XP055107654, ISSN: 0021-8820, DOI: 10.1038/ja.2010.5

## Description

### Field of the invention

The present invention generally relates to the field of pesticides. More specifically, the present invention relates to novel spinosyn compounds having advantageous properties, processes for their preparation, and their use as pesticides.

### Background of the invention

Spinosyns are a large family of natural compounds produced through fermentation of several species of soil bacteria of the genus *Saccharopolyspora.* Multiple structurally diverse spinosyn analogs are biosynthesized concurrently and can be isolated from fermentation broths of producing organisms, for example Saccharopolyspora spinosa, Saccharopolyspora pogona and Saccharopolyspora ASAGF58. Each isolated analog is given a generic name of a "spinosyn" compound, according to S. spinosa, the first described producing organism. Spinosyns share a core structure, having a polyketide-derived tetracyclic macrolide core with two saccharide moieties linked through glycosidic bonds. Many of the naturally occurring analogs exhibit potent insecticidal activities against many commercially significant pest species that cause extensive damage to crops and other plants. Some of these analogs also exhibit activity against important external parasites of livestock, companion animals and of humans. Spinosyns have a unique mechanism of action (MOA) involving disruption of nicotinic acetylcholine receptors. When compared with many other insecticides, spinosyns generally show greater selectivity toward target insects and lesser activity against many of their beneficial predators.

Butenyl spinosyns are a subgroup of spinosyns, characterized by the butenyl side chain at the carbon 21 (C21) position of the macrolide core. They are produced by *S. pogona* (Hahn et al. (2006); EP1373290) and *Saccharopolyspora* ASAGF58 (Guo et al. (2020)). Several butenyl spinosyns, among them butenyl spinosyn α1 were shown to have comparable or even superior insecticidal activity, compared to spinosyn A, the main component of a commercial product Spinosad, produced by S. spinosa (Lewer et al. (2009)). Possibly, superior activity is caused by a more hydrophobic nature of the C21 butenyl side chain compared to C21 ethyl moiety, present in classical spinosyns, such as spinosyn A.

Numerous semi-synthetic spinosyn derivatives were generated in the past, based either on the most abundant natural compounds spinosyn A and spinosyn D or on less abundant natural factors or on compounds, produced by mutants of the producing organisms, such as Saccharopolyspora spinosa (EP0837870, US10570166, WO2017/040878). Butenyl spinosyns were also subjected to further chemical modification, generating numerous semi-synthetic derivatives (EP 1370566). In one study, the possibilities to derivatize the double bond of the C21 butenyl-side chain were explored (Daeuble et al. (2009)). In addition, derivatives based on the ring-expanded 14-membered lactone and C-8 hydroxyl group were produced. In contrast, many of the other sites within the spinosyn/butenyl spinosyn aglycone had been inert to chemical modification because of their saturated hydrocarbon nature. CN 102190694 A discloses spinosyn compounds having insecticidal activity. CN 111632061 A discloses spinosyn compounds having arginine succinate synthase activity. WO 2020/023943 discloses formulations of spinosyn compounds for the treatment of an ocular Demodex mite infestation. US 6919464 B1 discloses derivatives of 21-butenyl spinosyns.

While some of the semi-synthetic derivatives were found to have interesting properties and structure-activity relationships were studied (Kirst (2010)), butenyl spinosyn or its derivatives have so far not been developed as commercial insecticidal products. In contrast, one semi-synthetic derivative, based on C21-ethyl spinosyns, named spinetoram, was developed as a commercial product (Kirst (2010)).

Spinosyns are known to be poorly soluble in water, which complicates the development of liquid formulations. Mixtures of surfactants/emulsifiers, organic solvents and adjuvants have to be added in order to assure adequate solubility of the active compounds. Therefore, novel spinosyn derivatives with improved solubility are needed to simplify formulation development and reduce the negative environmental impacts of these additives on the environment and production cost. In order to increase spinosyn solubility weakly acidic solutions are prepared or addition salts with tartrate ions are prepared (DeAmicis et al. (1997)), resulting in protonated form of the dimethylamino group on the forosamine sugar. Several quaternary ammonium salts of spinosyns have been reported, however, such modification was shown to change the biological activity of spinosyn compounds towards anti-protozoan, anti-viral and anti-cancer activities (WO 2010150100, Ma et al. (2018)). Therefore, such compounds are expected to be less specific and in general less useful as insecticides for use in agriculture.

With growing concerns over the effects of pesticides on aquatic life and beneficial insects (Ramachanderan and Schaefer (2020)) such as pollinators, and inevitable occurrence of resistance among target pests, there is a need to develop and bring to market novel members of the spinosyn family, particularly of the so far neglected butenyl spinosyn class. Preferably, novel molecules should also offer advantages in terms of formulation development.

Against this background, it is an object of the present invention to provide structurally novel spinosyn compounds having advantageous properties.

### Summary of the invention

The present invention provides novel spinosyn compounds having advantageous properties. More specifically, the present invention provides novel prodrugs of spinosyn compounds capable of releasing the active spinosyn compound differentially in a pH dependent manner. Advantageously, the spinosyn prodrugs of the present invention release the active compounds preferentially at high pH values, such as pH>7, pH>9 or pH>10. Additionally, the spinosyn compounds of the present invention are degraded at low pH values, such as pH<4. The spinosyn compounds of the present invention also show increased solubility in aqueous media, compared to standard spinosyn compounds, particularly in aqueous media with high pH, such as pH>6 or pH>7. Lastly, the spinosyn compounds of the present invention exhibit better efficacy and enable simplified formulations compared to classical spinosyn compounds.

The present invention provides in a main aspect a compound of general formula (II) 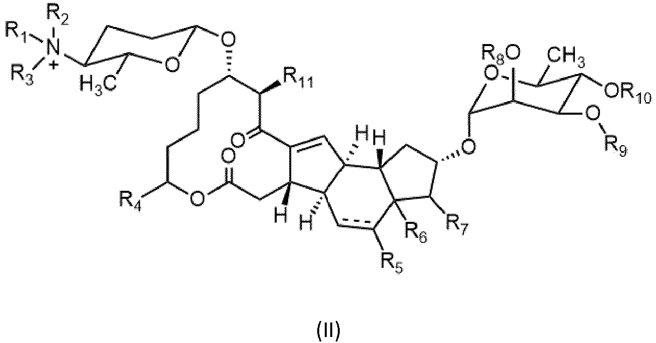 wherein
the dashed line is a single bond, a double bond or an epoxide;
R₁ is *-(CH₂)n-O-C(O)-R₁b, wherein
   * denotes an attachment to the nitrogen atom;
   n is an integer ranging from 1 to 10, or is an integer ranging from 1 to 5; and
   R₁b is selected from the group consisting of *-C(R₂b)₃, *-N(R₂b)₂, *-OH and *-OC(R₂b)₃, wherein
      * denotes an attachment to the carbonyl carbon atom; and
      each R₂b is independently selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₂ is selected from the group consisting of substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₃ is selected from the group consisting of substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₄ is selected from the group consisting of unsubsituted ethyl, unsubsituted propyl, unsubsituted butyl, unsubsituted 1-butenyl, unsubsituted 1,3-butadienyl and unsubsituted 3-hydroxy-1-butenyl, preferably is unsubsituted ethyl or unsubsituted 1-butenyl, more preferably is unsubsituted 1-butenyl;
R₅ is -H or unsubsituted methyl; preferably is -H;
R₆ is -H;
R₇ is -H or -OR_{7'}, wherein R_{7'} is hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₈ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl;
R₉ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl;
R₁₀ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl; and
R₁₁ is selected from the group consisting of hydrogen and substituted or unsubsituted C₁-C₂ alkyl; preferably is hydrogen or substituted or unsubstituted methyl; more preferably is hydrogen or unsubstituted methyl; most preferably is unsubstituted methyl,
in the form of a corresponding salt thereof.

More specifically, the present invention provides a compound according to claim 1, wherein the compound is a compound of general formula (III) 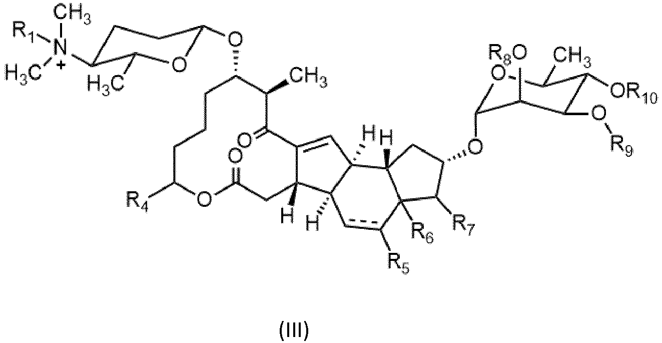 wherein
the dashed line is a single bond, a double bond or an epoxide;
R₁ is *-(CH₂)n-O-C(O)-R₁b, wherein
   * denotes an attachment to the nitrogen atom;
   n is an integer ranging from 1 to 10, or is an integer ranging from 1 to 5; and
   R₁b is selected from the group consisting of *-C(R₂b)₃, *-N(R₂b)₂, *-OH and *-OC(R₂b)₃, wherein
      * denotes an attachment to the carbonyl carbon atom; and
      each R₂b is independently selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₄ is selected from the group consisting of unsubsituted ethyl, unsubsituted propyl, unsubsituted butyl, unsubsituted 1-butenyl, unsubsituted 1,3-butadienyl and unsubsituted 3-hydroxy-1-butenyl, preferably is unsubsituted ethyl or unsubsituted 1-butenyl, more preferably is unsubsituted 1-butenyl;
R₅ is -H or unsubsituted methyl; preferably is -H;
R₆ is -H;
R₇ is -H or -OR_{7'}, wherein R_{7'} is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₈ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl;
R₉ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl; and
R₁₀ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl,
in the form of a corresponding salt thereof.

The present invention further provides a composition comprising a compound of the present invention and one or more physiologically acceptable adjuvants.

The present invention further provides the use of a compound of the present invention as a pesticide.

The present invention further provides a method for controlling a pest, such as a plant pest, comprising contacting a pest, such as a plant pest, with a compound of the present invention or a composition according to the present invention.

The present invention further provides a method for protecting a plant against a plant pest, comprising the step of: applying a compound of the present invention or a composition comprising the same and a carrier to a plant in need thereof.

The present invention is now described in more detail.

### Detailed description of the invention

The present invention provides novel spinosyn compounds having advantageous properties. More specifically, the spinosyn compounds of the present invention represent spinosyn prodrugs capable of releasing the active spinosyn compound differentially in a pH dependent manner. Advantageously, the provided spinosyn compounds release the active compounds preferentially at high pH values, such as pH>7, pH>9 or pH>10. Additionally, the provided compounds are degraded at low pH values, such as ph<4. The provided spinosyn compounds show increased solubility in aqueous media, compared to standard spinosyn compounds, particularly in aqueous media with high pH, such as pH>6. The provided spinosyn compounds exhibit better efficacy and enable simplified formulations compared to classical spinosyn compounds.

In one aspect, the present invention provides a compound of general formula (II) 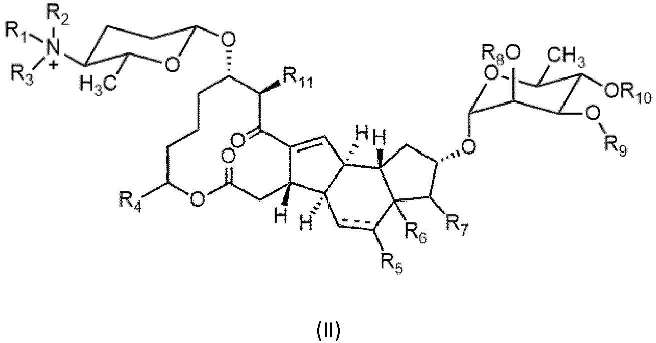 wherein
the dashed line is a single bond, a double bond or an epoxide;
R₁ is *-(CH₂)n-O-C(O)-R₁b, wherein
   * denotes an attachment to the nitrogen atom;
   n is an integer ranging from 1 to 10, or is an integer ranging from 1 to 5; and
   R₁b is selected from the group consisting of *-C(R₂b)₃, *-N(R₂b)₂, *-OH and *-OC(R₂b)₃, wherein
      * denotes an attachment to the carbonyl carbon atom; and
      each R₂b is independently selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₂ is selected from the group consisting of substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₃ is selected from the group consisting of substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₄ is selected from the group consisting of unsubsituted ethyl, unsubsituted propyl, unsubsituted butyl, unsubsituted 1-butenyl, unsubsituted 1,3-butadienyl and unsubsituted 3-hydroxy-1-butenyl, preferably is unsubsituted ethyl or unsubsituted 1-butenyl, more preferably is unsubsituted 1-butenyl;
R₅ is -H or unsubsituted methyl; preferably is -H;
R₆ is -H;
R₇ is -H or -OR_{7'}, wherein R_{7'} is hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₈ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl;
R₉ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl;
R₁₀ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl; and
R₁₁ is selected from the group consisting of hydrogen and substituted or unsubsituted C₁-C₂ alkyl; preferably is hydrogen or substituted or unsubstituted methyl; more preferably is hydrogen or unsubstituted methyl; most preferably is unsubstituted methyl,
in the form of a corresponding salt thereof.

According to some embodiments, the compound according to Formula (II) is a compound of general formula (III) 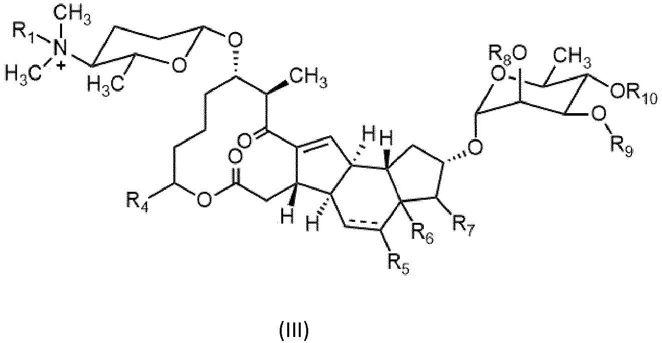 wherein
the dashed line is a single bond, a double bond or an epoxide;
R₁ is *-(CH₂)n-O-C(O)-R₁b, wherein
   * denotes an attachment to the nitrogen atom;
   n is an integer ranging from 1 to 10, or is an integer ranging from 1 to 5; and
   R₁b is selected from the group consisting of *-C(R₂b)₃, *-N(R₂b)₂, *-OH and *-OC(R₂b)₃, wherein
      * denotes an attachment to the carbonyl carbon atom; and
      each R₂b is independently selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₄ is selected from the group consisting of unsubsituted ethyl, unsubsituted propyl, unsubsituted butyl, unsubsituted 1-butenyl, unsubsituted 1,3-butadienyl and unsubsituted 3-hydroxy-1-butenyl, preferably is unsubsituted ethyl or unsubsituted 1-butenyl, more preferably is unsubsituted 1-butenyl;
R₅ is -H or unsubsituted methyl; preferably is -H;
R₆ is -H;
R₇ is -H or -OR_{7'}, wherein R_{7'} is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₈ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl;
R₉ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl; and
R₁₀ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl,
in the form of a corresponding salt thereof.

According to some embodiments, R₄ is unsubstituted ethyl or unsubstituted 1-butenyl and/or R₅ is -H and/or R₇ is -H.

According to some embodiments, each of R₈, R₉ and R₁₀ is substituted or unsubsituted C₁-C₆ alkyl.

According to some embodiments, each of R₈, R₉ and R₁₀ is -CH₃.

According to some embodiments, n is 1, 2, 3, 4 or 5.

According to some embodiments, R₁b is *-C(R₂b)₃ or *-OC(R₂b)₃.

According to some embodiments, each R₂b is independently selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₂ is substituted or unsubsituted C₁-C₆ alkyl and/or R₃ is substituted or unsubsituted C₁-C₆ alkyl.

According to some embodiments, R₁b is *-C(R₂b)₃.

According to some embodiments, R₁b is *-N(R₂b)₂.

According to some embodiments, R₁b is *-OH.

According to some embodiments, R₁b is *-OC(R₂b)₃.

According to some embodiments, each R₂b is independently selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, each R₂b is independently selected from the group consisting of hydrogen, substituted or unsubsituted C₁-C₆ alkyl, substituted or unsubsituted C₂-C₆ alkenyl and substituted or unsubsituted C₂-C₆ alkynyl.

According to some embodiments, each R₂b is independently selected from the group consisting of hydrogen and substituted or unsubsituted C₁-C₆ alkyl.

According to some embodiments, each R₂b is independently selected from the group consisting of hydrogen and unsubsituted C₁-C₆ alkyl.

According to some embodiments, each R₂b is independently selected from the group consisting of hydrogen and unsubsituted C₁-C₄ alkyl.

According to some embodiments, each R₂b is independently selected from the group consisting of hydrogen and -CH₃.

According to some embodiments, n is an integer ranging from 1 to 10.

According to some embodiments, n is an integer ranging from 1 to 5.

According to some embodiments, n is 1, 2, 3 or 4.

According to some embodiments, n is 1, 2 or 3.

According to some embodiments, n is 1 or 2.

According to some embodiments, n is 1.

According to some embodiments, n is 2.

According to some embodiments, n is 3.

According to some embodiments, n is 4.

According to some embodiments, n is 5.

According to some embodiments, R₂ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₂ is hydrogen or substituted or unsubsituted C₁-C₆ alkyl.

According to some embodiments, R₂ is substituted or unsubsituted C₁-C₆ alkyl.

According to some embodiments, R₂ is unsubsituted C₁-C₆ alkyl.

According to some embodiments, R₂ is unsubsituted C₁-C₂ alkyl.

According to some embodiments, R₂ is -CH₃.

According to some embodiments, R₃ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₃ is hydrogen or substituted or unsubsituted C₁-C₆ alkyl.

According to some embodiments, R₃ is substituted or unsubsituted C₁-C₆ alkyl.

According to some embodiments, R₃ is unsubsituted C₁-C₆ alkyl.

According to some embodiments, R₃ is unsubsituted C₁-C₂ alkyl.

According to some embodiments, R₃ is -CH₃.

According to some embodiments, R₄ is unsubstituted ethyl or unsubstituted 1-butenyl.

According to some embodiments, R₄ is unsubstituted 1-butenyl.

According to some embodiments, R₅ is -H.

According to some embodiments, R₅ is -CH₃.

According to some embodiments, R₇ is -H.

According to some embodiments, R₇ is OR_{7'}.

According to some embodiments, R_{7'} is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R_{7'} is selected from the group consisting of hydrogen, substituted or unsubsituted C₁-C₆ alkyl, substituted or unsubsituted C₂-C₆ alkenyl, and substituted or unsubsituted C₂-C₆ alkynyl.

According to some embodiments, R_{7'} is selected from the group consisting of hydrogen and substituted or unsubsituted C₁-C₆ alkyl.

According to some embodiments, R_{7'} is substituted or unsubsituted C₁-C₂ alkyl; preferably is -CH₃.

According to some embodiments, R₈ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₈ is selected from the group consisting of hydrogen, substituted or unsubsituted C₁-C₆ alkyl, substituted or unsubsituted C₂-C₆ alkenyl and substituted or unsubsituted C₂-C₆ alkynyl.

According to some embodiments, R₈ is hydrogen or substituted or unsubsituted C₁-C₆ alkyl.

According to some embodiments, R₈ is substituted or unsubsituted C₁-C₂ alkyl; preferably is -CH₃.

According to some embodiments, R₉ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₉ is selected from the group consisting of hydrogen, substituted or unsubsituted C₁-C₆ alkyl, substituted or unsubsituted C₂-C₆ alkenyl and substituted or unsubsituted C₂-C₆ alkynyl.

According to some embodiments, R₉ is hydrogen or substituted or unsubsituted C₁-C₆ alkyl.

According to some embodiments, R₉ is substituted or unsubsituted C₁-C₂ alkyl; preferably is -CH₃.

According to some embodiments, R₁₀ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₁₀ II) is selected from the group consisting of hydrogen, substituted or unsubsituted C₁-C₆ alkyl, substituted or unsubsituted C₂-C₆ alkenyl and substituted or unsubsituted C₂-C₆ alkynyl.

According to some embodiments, R₁₀ is hydrogen or substituted or unsubsituted C₁-C₆ alkyl.

According to some embodiments, R₁₀ is substituted or unsubsituted C₁-C₂ alkyl; preferably is -CH₃.

According to some embodiments, R₁₁ is selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

According to some embodiments, R₁₁ is selected from the group consisting of hydrogen, substituted or unsubsituted C₁-C₆ alkyl, substituted or unsubsituted C₂-C₆ alkenyl and substituted or unsubsituted C₂-C₆ alkynyl.

According to some embodiments, R₁₁ is hydrogen or substituted or unsubsituted methyl.

According to some embodiments, R₁₁ is hydrogen.

According to some embodiments, R₁₁ is substituted or unsubsituted methyl.

According to some embodiments, R₁₁ is -CH₃.

According to some embodiments, each of R₈, R₉ and R₁₀ is substituted or unsubsituted C₁-C₆ alkyl.

According to some embodiments, each of R₈, R₉ and R₁₀ is unsubsituted C₁-C₆ alkyl.

According to some embodiments, each of R₈, R₉ and R₁₀ is -CH₃.

According to some embodiments, the compound is selected from the group consisting of 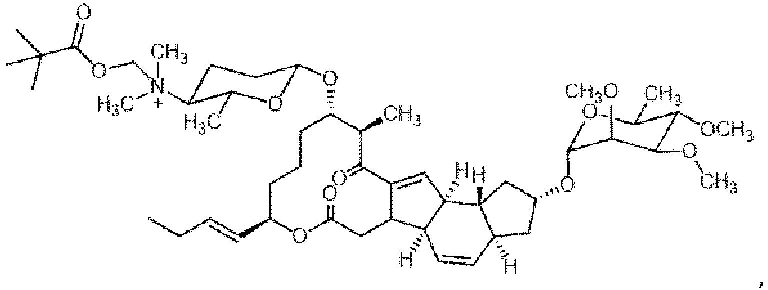 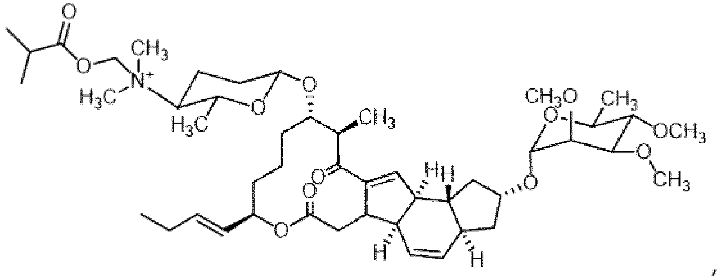 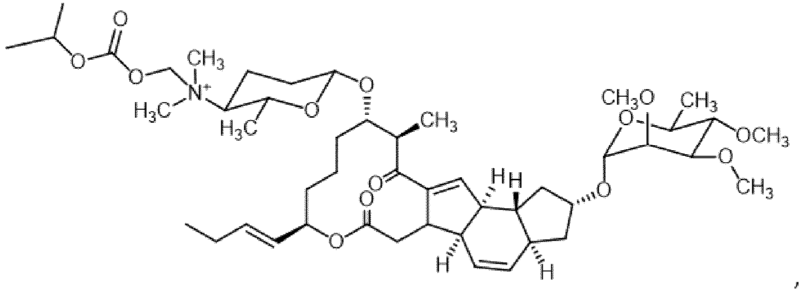 in the form of a corresponding salt thereof.

According to some embodiments, the corresponding salt is with an anion selected from the group consisting of chloride, sulfate, nitrate, phosphate, citrate, tartrate, acetate, lactate, propionate and gluconate.

The compounds of the present invention may be made by a process comprising reacting a compound of formula IIa 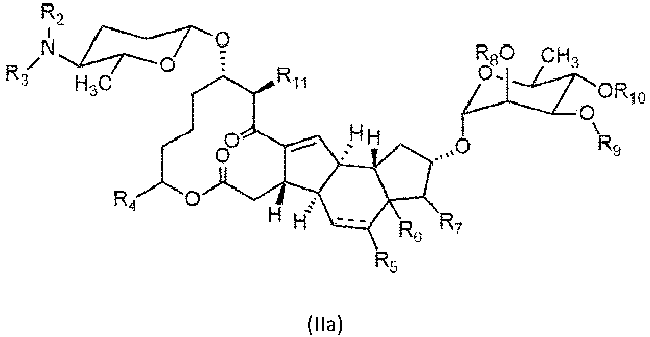 with a compound of formula IV 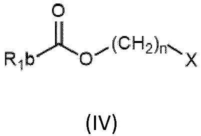 wherein X is a halogen, preferably Cl, Br or I, and R₁b, R₂, R₃, R₄, R₅, R₆, R₇, R₈, R₉, R₁₀ and R₁₁ being as defined herein.

The compounds of the present invention may be made by a process comprising reacting a compound of formula IIIa 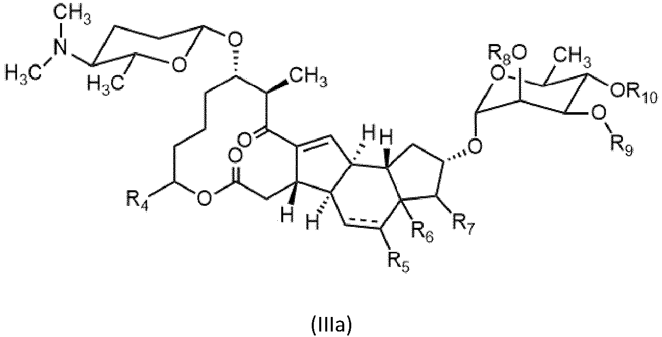 with a compound of formula IV 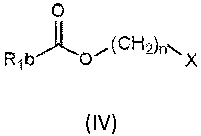 wherein X is a halogen, preferably Cl, Br or I, and R₁b, R₂, R₃, R₄, R₅, R₆, R₇, R₈, R₉ and R₁₀ being as defined herein.

If not commercially available, the necessary starting materials for the processes of the invention may be made by procedures which are selected from standard organic chemistry techniques, techniques which are analogous to the synthesis of known structurally similar compounds, or techniques, which are analogous to the procedures described in the examples.

The reaction between a compound of formula (Ila) or (Illa) and a compound of formula IV is preferably carried out in a suitable solvent, preferably a polar aprotic solvent such as acetonitrile, dimethylsulfoxide or dimethylformamide, most preferably acetonitrile, at a suitable temperature, such as between room temperature and the reflux temperature, preferably at a temperature ranging from 50°C to 60°C. Generally, the reaction may be carried out for any period of time suitable for the formation of a compound of the invention. Suitably, the reaction is carried out for at least 3 hours, such as from 3 to 6 hours. Optionally, the reaction can be carried out in the presence of organic or inorganic bases, preferably a non-nucleophilic, hindered base and a proton scavenger such as 1,2,2,6,6-pentamethylpiperidine, potassium carbonate or caesium carbonate, preferably 1,2,2,6,6-pentamethylpiperidine. The process may further comprise an purification step after completion of the reaction. The purification step may include any any conventional procedure for purification of chemical compounds from a reaction. Well-known purification procedures include centrifugation or filtration, precipitation, and chromatographic methods such as e.g. ion exchange chromatography, gel filtration chromatography, etc.

The present invention further provides a composition comprising a compound according the present invention and one or more physiologically acceptable adjuvants.

Composition of the present invention include concentrated versions, in which the present active agent is present in a concentration of from 0.001 to 98.0 percent, with the remaining content being physiologically acceptable carriers. Such compositions, especially those with less than 50 percent of the present compound, can sometimes be used directly, but these compositions can also be diluted with other physiologically acceptable carriers to form more dilute treating formulations. These latter compositions can include the active agent in lesser concentrations of from 0.001 to 0.1 percent. Compositions are prepared according to the procedures and formulas which are conventional in the agricultural or pest control art. The compositions may be concentrated and dispersed in water or may be used in the form of a dust, bait or granular formulation. The dispersions are typically aqueous suspensions or emulsions prepared from concentrated formulations of the compounds. The watersoluble or water-suspension or emulsifiable formulations are either solids, wettable powders, or liquids, known as emulsifiable concentrates or aqueous suspensions. Wettable powders may be agglomerated or compacted to form water dispersible granules. These granules comprise mixtures of compound, inert carriers and surfactants. The concentration of the compound is typically between about 0 .1% to about 90% by weight. The inert carrier is typically attapulgite clays, montmorillonite clays and the diatomaceous earths or purified silicates. Surfactants comprise typically about 0.5% to about 10% of the wettable powder. Surfactants include sulfonated lignins, condensed napthalenesulfonates, the napthalenesulfonates, alkyl-benenesulfonates, alkysulfonates or nonionic surfactants such as ethylene oxide adducts of alkylphenols or mixtures thereof. Emulsifiable concentrates of the derivatives of the invention typically range from about 50 to about 500 grams of spinosyn derivative per liter of liquid, equivalent to about 10% to about 50%, dissolved in an inert carrier which is a mixture of a water immiscible solvent and emulsifiers. Organic solvents include organics such as xylenes, and petroleum fractions such as high-boiling naphthlenic and olefinic portions of petroleum which include heavy and aromatic naphtha. Other organics may also be used such as terpenic solvents -rosin derivatives, aliphatic ketones such as cyclohexanone and complex alcohols. Emulsifiers for emulsifiable concentrates are typically mixed ionic and/or nonionic surfactants such as those mentioned herein or their equivalents. Aqueous suspensions may be prepared containing water-insoluble spinosyn derivatives, where the compounds are dispersed in an aqueous vehicle at a concentration typically in the range of between about 5% to about 50% by weight. The suspensions are prepared by finely grinding the compound and vigorously mixing it into a vehicle of water, surfactants, and dispersants. Inert ingredients such as inorganic salts and synthetic or natural gums may also be employed to increase the density and/or viscosity of the aqueous vehicle as is desired. Precipitated flowables may be prepared by dissolving at least one spinosyn derivative of the invention in a water-miscible solvent and surfactants or surface active polymers. When these formulations are mixed with water, the active spinosyn derivative precipitates with the surfactant controlling the size of the resulting micro-crystalline precipitate. The size of the crystal can be controlled through the selection of specific polymer and surfactant mixtures. The spinosyn derivatives may also be applied as a granular composition that is applied to the soil. The granular composition typically contains from about 0.5% to about 10% by weight of the derivative. The spinosyn derivative is dispersed in an inert carrier which is typically clay or an equivalent substance. Generally, granular compositions are prepared by dissolving the compounds of the invention in a suitable solvent and applying it to a granular carrier which has been pre-formed to the desirable particle size. The particle size is typically between about 0.5 mm to 3 mm. The granular compositions may also be prepared by forming a dough or paste of the carrier and compound, drying the combined mixture, and crushing the dough or paste to the desired particle size.

The spinosyn derivative may also be combined with an appropriate organic solvent. The organic solvent is typically a bland petroleum oil that is widely used in the agricultural industry. These combinations are typically used as a spray. More typically, the spinosyn compounds are applied as a dispersion in a liquid carrier, where the liquid carrier is water. The compounds may also be applied in the form of an aerosol composition. The compound is dissolved in an inert carrier, which is a pressure-generating propellant mixture. The aerosol composition is packaged in a container, where the mixture is dispersed through an atomizing valve. Propellant mixtures contain either low-boiling halocarbons, which may be mixed with organic solvents or aqueous suspensions pressurized with inert gases or gaseous hydrocarbons. The compounds may be applied to any locus inhabited by an insect or mite. Such locus typically is cotton, soybean and vegetable crops, fruit and nut trees, grape vines, houses and ornamental plants. The amount of the spinosyn derivative applied to the loci of insects and mites can be determined by those skilled in the art. Generally, the concentrations of from about 10 ppm to about 5,000 ppm provide the desired control. For crops such as soybeans and cotton, the rate of application is about 0.01 to about 1 kg/ha, where the spinosyn derivative is applied in a 5 to 50 gal/A spray formulation.

The composition can be formulated in a liquid concentrate, ready-to-use (RTU) liquid spray, dust, or solid form. The formulation chosen will depend on the use of the product. The following general treatment methods are preferably suitable for carrying out the seed treatment, or plant propagation material treatment, according to the invention: dry treatments (preferably with addition of adhesion promoters such as, for example, liquid paraffin or talc), and, if appropriate, colorants, slurry treatments (preferably with addition of wetters, dispersants, emulsifiers, adhesives, inert fillers and colorants), aqueous liquid treatments (preferably with addition of emulsifiers, dispersants, thickeners, antifreeze agents, polymers, adhesives and colorants), solvent-based liquid treatments (with addition of solvents and colorants), emulsion treatments (with addition of emulsifiers, solvents and colorants).

The present invention further provides the use of a compound or composition according to the present invention as a pesticide.

The compounds according to the present invention or a composition according to the present invention may also be used as an insecticide.

The present invention further provides the use of a compound or composition according to the present invention in controlling a pest, such as a plant pest.

The present invention further provides the use of a compound or composition according to the present invention in protecting a plant against a plant pest.

According to some embodiments, the compound or the composition is used in protecting a plant against a plant pest during flowering of said plant.

According to some embodiments, the compound or the composition of the present invention may be used in protecting a plant against a plant pest during pollination by a bee, such as a honey bee.

The present invention further provides a method for controlling a pest, such as a plant pest, comprises contacting a pest, such as a plant pest, with a compound or composition of the present invention.

The present invention further provides a method for protecting a plant against a plant pest, comprising the step of: applying a compound of the present invention or a composition of the present invention to a plant in need thereof.

Being initially a non-active spinosyn derivative, the compound of the present invention becomes converted into an active spinosyn compound through a chemical reaction caused by alkaline conditions in the pest's digestive system once intaken by the pest.

Thus a method for releasing an active spinosyn compound form the compound of the present invention comprises exposing the compound of the present invention to a solution with alkaline pH value.

The present invention further provides a method for protecting a plant against a plant pest, comprising the step of: applying a compound or composition of the present invention to a plant in need thereof.

The compound or composition as defined above may be used on any plant in need of being protected against a plant pest. The plant may be an angiosperm or gymnosperm. According to some embodiments, the plant is an angiosperm. According to some embodiment, the plant is a gymnosperm. The plant may be a dicot or monocot. According to some embodiments, the plant is a dicot. According to some embodiment, the plant is a monocot. The plant may be a food plant (i.e. a plant some parts of which provides food for animal or human consumption), such as fruit plant.

The plant may be a crop plant, such as a food crop plant. According to certain embodiments, the plant is a food crop plant selected from the group consisting of pepper plant, cocoa plant, tomato plant, potato plant, maize plant, wheat plant and rice plant. The plant may be a tobacco plant, such as *Nicotiana tabacum.*

The plant pest can be an insect, such as a herbivorous insect, an arachnid or a nematode. Therefore, according to certain embodiments, the plant pest is an insect. According to some embodiments, the plant pest is a herbivorous insect.

According to some embodiments, the insect is of the order *Coleoptera* or *Lepidoptera.*

According to some embodiments, the insect is of the order *Coleoptera.*

According to some embodiments, the insect is of the order *Lepidoptera.*

According to some embodiments, the insect is of the family Chrysomelidae, *Crambidae, Plutellidae, Gelechiidae, Gracillariidae, Tortricidae, Noctuidae, Pyralidae* or *Pieridae.*

According to some embodiments, the insect is of the genus *Leptinotarsa, Chilo, Cnaphalocrocis, Plutella, Tuta, Chrysodeixis, Anarsia, Diatraea, Lobesia, Eupoecillia, Phyllocnistis, Diabrotica, Tortrix, Cydia, Spodoptera, Helicoverpa, Ostrinia* or *Pieris*

According to some embodiments, the insect is *Leptinotarsa decemlineata, Plutella xylostella, Tuta absoluta, Chrysodeixis includens, Cydia pomonella, Anarsic lineatella, Diatraea venostata, Lobesia botrana, Eupoecillia ambliguella, Phyllocnistis citrella, Diabrotica virgifera virgifera, Cydia pomonella, Helicoverpa zea, Peridroma saucia, Ostrinia nubilialis* or *Pieris brassicae.*

The plant pest may be a larva or an imago of the insect. According to some embodiments, the plant pest is a larva of the insect. The larva may be in any stage of larval development, such as a larval stage selected from the group consisting of L1, L2, L3, L4, and L5. According to some embodiments, the plant pest is an imago of the insect.

The compound of the present invention or composition according to the present invention may be used in agriculture.

Generally, the compound or composition can be applied to a plant in need thereof in any suitable dose, frequency and method of administration.

The compound or composition may suitably be in liquid form, and may be applied by spraying, drenching or dropping onto the plant. According to some embodiments, the compound or composition is applied by drenching. According to some embodiments, compound or composition is applied by spraying. According to some embodiments, the composition is applied by dropping.

The compound, either as raw material or in the form of a composition, may be applied at any effective amount, for example, at a concentration ranging from about 0.1 µM to about 100 mM. Generally, a effective amount is one at which the active spinosyn compound shows insecticidal activity. The effective amount may thus vary depending on the actual active spinosyn compound employed and can be determined by the skilled person.

The compound or composition may be applied at least once a week. For example, it may be applied 1 to 3 times a week, such as two times a week. The compound may be applied at least once a day. For example, it may be applied 1 to 3 times a day, such as twice a day.

### Certain definitions

In the context of this invention, a "pesticide" is a compound or composition that is meant to control a plan pests, and includes insecticide, nematicide and molluscicide.

In the context of this invention, a "insecticide" is a compound or composition used for reducing or eliminating insects harmful to cultivated plants.

In the context of this invention, "alkyl" is understood as meaning saturated, linear or branched hydrocarbons, which may be unsubstituted or substituted once or several times. It encompasses e.g. -CH₃ and -CH₂-CH₃. In these radicals, C₁-C₂ alkyl represents C1- or C2-alkyl, C₁-C₃ alkyl represents C1-, C2- or C3-alkyl, C₁-C₄ alkyl represents C1-, C2-, C3- or C4-alkyl, C₁-C₅ alkyl represents C1-, C2-, C3-, C4-, or C5-alkyl, C₁-C₆ alkyl represents C1-, C2-, C3-, C4-, C5- or C6-alkyl, C₁-C₇ -alkyl represents C1-, C2-, C3-, C4-, C5-, C6- or C7-alkyl, C₁-C₈ alkyl represents C1-, C2-, C3-, C4-, C5-, C6-, C7- or C8-alkyl, C₁-C₁₀ alkyl represents C1-, C2-, C3-, C4-, C5-, C6-, C7-, C8-, C9- or C10-alkyl, C₁-C₁₈ -alkyl represents C1-, C2-, C3-, C4-, C5-, C6-, C7-, C8-, C9-, C10-, C11-, C12-, C13-, C14-, C15-, C16-, C17- or C18-alkyl, and C₁-C₂₀ - alkyl represents C1-, C2-, C3-, C4-, C5-, C6-, C7-, C8-, C9-, C10-, C11-, C12-, C13-, C14-, C15-, C16-, C17-, C18-, C19 or C20-alkyl. The alkyl radicals are preferably methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc. Preferably alkyl is understood in the context of this invention as C₁-C₈ alkyl like methyl, ethyl, propyl, butyl, pentyl, hexyl, heptyl, or octyl; more preferably as C₁-C₆ alkyl like methyl, ethyl, propyl, butyl, pentyl, or hexyl; and most preferably as C₁-C₄ alkyl like methyl, ethyl, propyl or butyl.

"Alkenyl" is understood as meaning unsaturated, linear or branched hydrocarbons, which may be unsubstituted or substituted once or several times. It encompasses groups like e.g. -CH=CH-CH₃. The alkenyl radicals are preferably vinyl (ethenyl), allyl (2-propenyl). Preferably in the context of this invention alkenyl is C₁-C₁₀ alkenyl or C₁-C₈ alkenyl like ethylene, propylene, butylene, pentylene, hexylene, heptylene or octylene; or is C₁-C₆ alkenyl like ethylene, propylene, butylene, pentylene, or hexylene; or is C₁-C₄ alkenyl, like ethylene, propylene, or butylenes.

"Alkynyl" is understood as meaning unsaturated, linear or branched hydrocarbons, which may be unsubstituted or substituted once or several times. It encompasses groups like e.g. -C=C-CH₃ (1-propinyl). Preferably alkynyl in the context of this invention is C₁-C₁₀ alkynyl or C₂₋₈-alkynyl like ethyne, propyne, butyene, pentyne, hexyne, heptyne, or octyne; or is C₁-C₆ alkynyl like ethyne, propyne, butyene, pentyne, or hexyne; or is C₁-C₄ alkynyl like ethyne, propyne, butyene, pentyne, or hexyne.

In connection with alkyl (also in alkylaryl, alkylheterocyclyl or alkylcycloalkyl), alkenyl and alkynyl - unless defined otherwise - the term "substituted" in the context of this invention is understood as meaning replacement of at least one hydrogen radical on a carbon atom by halogen (F, Cl, Br, I), cyano, hydroxy, amino or carboxyl. More than one replacement on the same molecule and also on the same carbon atom is possible with the same or different substituents. This includes for example 3 hydrogens being replaced on the same C atom, as in the case of CF₃, or at different places of the same molecule, as in the case of e.g. -CH(OH)-CH=CH-CHCl₂.

In the context of this invention "haloalkyl" is understood as meaning an alkyl being substituted once or several times by a halogen (selected from F, Cl, Br, I). It encompasses e.g. -CH₂Cl, -CH₂F, -CHCl₂, - CHF₂, -CCl₃, -CF₃ and -CH₂-CHCl₂. Preferably haloalkyl is understood in the context of this invention as halogen-substituted C₁₋₄-alkyl representing halogen substituted C1-, C2-, C3- or C4-alkyl. The halogen-substituted alkyl radicals are thus preferably methyl, ethyl, propyl, and butyl. Preferred examples include -CH₂Cl, -CH₂F, -CHCl₂, -CHF₂, and -CF₃.

In the context of this invention "haloalkoxy" is understood as meaning an -O-alkyl being substituted once or several times by a halogen (selected from F, Cl, Br, I). It encompasses e.g. -OCH₂Cl, -OCH₂F, - OCHCl₂, -OCHF₂, -OCCl₃, -OCF₃ and -OCH₂-CHCl₂. Preferably haloalkyl is understood in the context of this invention as halogen-substituted -O-C₁-C₄ alkyl representing halogen substituted C1-, C2-, C3- or C4-alkoxy. The halogen-substituted alkyl radicals are thus preferably O-methyl, O-ethyl, O-propyl, and O-butyl. Preferred examples include -OCH₂Cl, -OCH₂F, -OCHCl₂, -OCHF₂, and -OCF₃.

In the context of this invention "cycloalkyl" is understood as meaning saturated and unsaturated (but not aromatic) cyclic hydrocarbons (without a heteroatom in the ring), which can be unsubstituted or once or several times substituted. Furthermore, C₃-C₁₂ cycloalkyl represents C3-, C4-, C5-, C6-, C7- , C8-, C9-, C10-, C11- or C12-cycloalkyl, C₃-C₄ cycloalkyl represents C3- or C4-cycloalkyl, C₃-C₅ cycloalkyl represents C3-, C4- or C5-cycloalkyl, C₃-C₆ cycloalkyl represents C3-, C4-, C5- or C6-cycloalkyl, C₃-C₇ cycloalkyl represents C3-, C4-, C5-, C6- or C7-cycloalkyl, C₃-C₈ cycloalkyl represents C3-, C4-, C5-, C6-, C7- or C8-cycloalkyl, C₄-C₅ cycloalkyl represents C4- or C5-cycloalkyl, C₄-C₆ cycloalkyl represents C4-, C5- or C6-cycloalkyl, C₄-C₇ cycloalkyl represents C4-, C5-, C6- or C7-cycloalkyl, C₅-C₆ cycloalkyl represents C5- or C6-cycloalkyl and C₅-C₇ cycloalkyl represents C5-, C6- or C7-cycloalkyl. Examples are cyclopropyl, 2-methylcyclopropyl, cyclopropylmethyl, cyclobutyl, cyclopentyl, cyclopentylmethyl, cyclohexyl, cycloheptyl, cyclooctyl, and also adamantly. Preferably in the context of this invention cycloalkyl is C₃-C₈ cycloalkyl like cyclopropyl, cyclobutyl, cyclopentyl, cyclohexyl, cycloheptyl, or cyclooctyl; or is C₃-C₇ cycloalkyl like cyclopropyl, cyclobutyl, cyclopentyl, cyclohexyl, or cycloheptyl; or is C₃-C₆ cycloalkyl like cyclopropyl, cyclobutyl, cyclopentyl or cyclohexyl, especially cyclopentyl or cyclohexyl.

"Aryl" is understood as meaning 3 to 12 membered mono or polycyclic ring systems with at least one aromatic ring but without heteroatoms even in only one of the rings. Examples are phenyl, naphthyl, fluoranthenyl, fluorenyl, tetralinyl or indanyl, 9H-fluorenyl or anthracenyl radicals, which can be unsubstituted or once or several times substituted. Preferably, the aryl is a monocyclic aryl. More preferably the aryl is a 5, 6 or 7 membered monocyclic aryl. Even more preferably the aryl is a 5 or 6 membered monocyclic aryl. Most preferably aryl is understood in the context of this invention as phenyl, naphtyl or anthracenyl, preferably is phenyl.

A "heterocyclyl" radical or group (also called heterocyclyl hereinafter) is understood as meaning 3 to 12 membered mono or polycyclic heterocyclic ring systems, with at least one saturated or unsaturated ring which contains one or more heteroatoms from the group consisting of nitrogen, oxygen and/or sulfur in the ring. A heterocyclic group can also be substituted once or several times.

Examples include non-aromatic heterocyclyls such as tetrahydropyrane, oxazepane, morpholine, piperidine, pyrrolidine as well as heteroaryls such as furan, benzofuran, thiophene, benzothiophene, pyrrole, pyridine, pyrimidine, pyrazine, quinoline, isoquinoline, phthalazine, thiazole, benzothiazole, indole, benzotriazole, carbazole and quinazoline.

Subgroups inside the heterocyclyls as understood herein include heteroaryls and non-aromatic heterocyclyls:
- the heteroaryl (being equivalent to heteroaromatic radicals or aromatic heterocyclyls) is an aromatic 3 to 12 membered mono or polycyclic heterocyclic ring system of one or more rings of which at least one aromatic ring contains one or more heteroatoms from the group consisting of nitrogen, oxygen and/or sulfur in the ring; preferably is an aromatic 3 to 12 membered mono or polycyclic heterocyclic ring system of one or two rings of which at least one aromatic ring contains one or more heteroatoms from the group consisting of nitrogen, oxygen and/or sulfur in the ring, more preferably is selected from furan, benzofuran, thiophene, benzothiophene, pyrrole, pyridine, pyrimidine, pyrazine, quinoline, isoquinoline, phthalazine, benzothiazole, indole, benzotriazole, carbazole, quinazoline, thiazole, imidazole, pyrazole, oxazole, thiophene and benzimidazole;
- the non-aromatic heterocyclyl is a 3 to 12 membered mono or polycyclic heterocyclic ring system of one or more rings of which at least one ring - with this (or these) ring(s) then not being aromatic - contains one or more heteroatoms from the group consisting of nitrogen, oxygen and/or sulfur in the ring; preferably is a 3 to 12 membered mono or polycyclic heterocyclic ring system of one or two rings of which one or both rings - with this one or two rings then not being aromatic - contain/s one or more heteroatoms from the group consisting of nitrogen, oxygen and/or sulfur in the ring, more preferably is selected from oxazepam, pyrrolidine, piperidine, piperazine, tetrahydropyran, morpholine, indoline, oxopyrrolidine, benzodioxane, oxetane, especially is benzodioxane, morpholine, tetrahydropyran, piperidine, oxopyrrolidine, oxetane and pyrrolidine.

Preferably, the heteroaryl is a monocyclic heteroaryl. More preferably the heteroaryl is a 5, 6 or 7 membered monocyclic heteroaryl. Even more preferably the heteroaryl is a 5 or 6 membered monocyclic heteroaryl.

Preferably, the non-aromatic heterocyclyl is a monocyclic non-aromatic heterocyclyl. More preferably the non-aromatic heterocyclyl is a 4, 5, 6 or 7 membered monocyclic non-aromatic heterocyclyl. Even more preferably the non-aromatic heterocyclyl is a 5 or 6 membered monocyclic non-aromatic heterocyclyl.

Preferably in the context of this invention "heterocyclyl" is defined as a 5 to 10 membered mono or polycyclic heterocyclic ring system of one or two saturated or unsaturated rings of which at least one ring contains one or more heteroatoms from the group consisting of nitrogen, oxygen and/or sulfur in the ring.

Preferred examples of heterocyclyls include oxetane, oxazepan, pyrrolidine, imidazole, oxadiazole, tetrazole, pyridine, pyrimidine, piperidine, piperazine, benzofuran, benzimidazole, indazole, benzodiazole, thiazole, benzothiazole, tetrahydropyrane, morpholine, indoline, furan, triazole, isoxazole, pyrazole, thiophene, benzothiophene, pyrrole, pyrazine, pyrrolo[2,3b]pyridine, quinoline, isoquinoline, phthalazine, benzo-1,2,5-thiadiazole, indole, benzotriazole, benzoxazole oxopyrrolidine, pyrimidine, benzodioxolane, benzodioxane, carbazole and quinazoline, especially is pyridine, pyrazine, indazole, benzodioxane, thiazole, benzothiazole, morpholine, tetrahydropyrane, pyrazole, imidazole, piperidine, thiophene, indole, benzimidazole, pyrrolo[2,3b]pyridine, benzoxazole, oxopyrrolidine, pyrimidine, oxazepane, oxetane and pyrrolidine.

In connection with aromatic heterocyclyls (heteroaryls), non-aromatic heterocyclyls, aryls and cycloalkyls, when a ring system falls within two or more of the above cycle definitions simultaneously, then the ring system is defined first as an aromatic heterocyclyl (heteroaryl) if at least one aromatic ring contains a heteroatom. If no aromatic ring contains a heteroatom, then the ring system is defined as a non-aromatic heterocyclyl if at least one non-aromatic ring contains a heteroatom. If no non-aromatic ring contains a heteroatom, then the ring system is defined as an aryl if it contains at least one aryl cycle. If no aryl is present, then the ring system is defined as a cycloalkyl if at least one non-aromatic cyclic hydrocarbon is present.

In the context of this invention "alkylaryl" is understood as meaning an aryl group (see above) being connected to another atom through a C₁₋₆-alkyl (see above) which may be branched or linear and is unsubstituted or substituted once or several times. Preferably alkylaryl is understood as meaning an aryl group (see above) being connected to another atom through 1 to 4 (-CH₂-) groups. Most preferably alkylaryl is benzyl (i.e. -CH₂-phenyl).

In the context of this invention "alkylheterocyclyl" is understood as meaning an heterocyclyl group being connected to another atom through a C₁₋₆-alkyl (see above) which may be branched or linear and is unsubstituted or substituted once or several times. Preferably alkylheterocyclyl is understood as meaning an heterocyclyl group (see above) being connected to another atom through 1 to 4 (-CH₂-) groups. Most preferably alkylheterocyclyl is -CH₂-pyridine.

In the context of this invention "alkylcycloalkyl" is understood as meaning an cycloalkyl group being connected to another atom through a C₁₋₆-alkyl (see above) which may be branched or linear and is unsubstituted or substituted once or several times. Preferably alkylcycloalkyl is understood as meaning an cycloalkyl group (see above) being connected to another atom through 1 to 4 (-CH₂-) groups. Most preferably alkylcycloalkyl is -CH₂-cyclopropyl.

Preferably, the cycloalkyl is a monocyclic cycloalkyl. More preferably the cycloalkyl is a 3, 4, 5, 6, 7 or 8 membered monocyclic cycloalkyl. Even more preferably the cycloalkyl is a 3, 4, 5 or 6 membered monocyclic cycloalkyl.

In connection with aryl (including alkyl-aryl), cycloalkyl (including alkyl-cycloalkyl), or heterocyclyl (including alkyl-heterocyclyl), substituted is understood - unless defined otherwise - as meaning substitution of the ring-system of the aryl or alkyl-aryl, cycloalkyl or alkyl-cycloalkyl; heterocyclyl or alkyl-heterocyclyl with one or more of halogen (F, Cl, Br, I), cyano, hydroxy, amino, carboxyl, haloalkyl, haloalkoxy, linear or branched, substituted or unsubstituted C₁₋₆-alkyl; a saturated or unsaturated, linear or branched, substituted or unsubstituted C₁₋₆-alkyl; a saturated or unsaturated, linear or branched, substituted or unsubstituted -O-C₁₋₆₋alkyl (alkoxy); a saturated or unsaturated, linear or branched, substituted or unsubstituted -S-C₁₋₆₋alkyl; a saturated or unsaturated, linear or branched, substituted or unsubstituted -C(O)-C₁₋₆₋alkyl-group; a saturated or unsaturated, linear or branched, substituted or unsubstituted -C(O)-O-C₁₋₆₋alkyl-group; -NH-Boc and -Boc. Preferably, suitable substituents are 1, 2 or 3 substituents independently selected from fluoro, chloro, bromo, iodo, cyano, nitro, amino, carboxyl, methylamino, dimethylamino, hydroxy, methyl, ethyl, methoxy, methylthio, methylsulfinyl, methylsulfonyl, phenyl, -NH-Boc and -Boc.

Additionally to the above-mentioned substitutions, in connection with cycloalkyl (including alkyl-cycloalkyl), or heterocycly (including alkylheterocyclyl) namely non-aromatic heterocyclyl (including non-aromatic alkyl-heterocyclyl), substituted is also understood - unless defined otherwise - as meaning substitution of the ring-system of the cycloalkyl or alkyl-cycloalkyl; non-aromatic heterocyclyl or non aromatic alkyl-heterocyclyl with 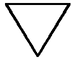 or =O.

A ring system is a system consisting of at least one ring of connected atoms but including also systems in which two or more rings of connected atoms are joined with "joined" meaning that the respective rings are sharing one (like a spiro structure), two or more atoms being a member or members of both joined rings.

The term "salt" is to be understood as meaning any form of the compound according to the invention in which it assumes a cationic form and is coupled with a counter-ion (i.e. an anion) or is in solution. By this are also to be understood complexes of the compound with other molecules and ions, in particular complexes via ionic interactions.

Salts can be formed with anions or acids and in the context of this invention is understood as meaning salts of at least one of the compounds according to the invention with at least one, preferably inorganic, anion. Non-limiting examples of an anion include chloride, sulfate, nitrate, phosphate, citrate, tartrate, acetate, lactate, propionate, gluconate and others.

Where a numerical limit or range is stated herein, the endpoints are included. Also, all values and sub ranges within a numerical limit or range are specifically included as if explicitly written out.

Having generally described this invention, a further understanding can be obtained by reference to certain specific examples, which are provided herein for purposes of illustration only, and are not intended to be limiting unless otherwise specified.

### Examples

### Example 1: Synthesis of compound J1A

Butenyl spinosyn α1 (800 mg, 1.06 mmol) and iodomethyl 2,2-dimethylpropanoate were dissolved in acetonitrile (16 mL) and the mixture was stirred at 50 °C for 3 hours. The reaction mixture was filtered to obtain crude product which was purified by reversed phase chromatography. pH of the eluted fractions was adjusted to pH 7 and lyophilized to give a white solid. The solid was suspended in acetonitrile (10 mL) and filtered to remove ammonium chloride. The filtrate was lyophilized to give J1A (395 mg, 41.2% yield, 98.8% purity) as a white solid. The correct structure of the compound was determined by LC/MS and ¹H NMR.

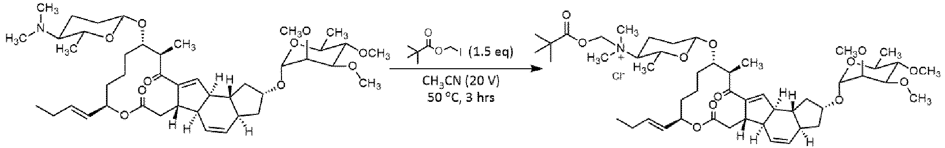

Analytical data for compound J1A:
¹H NMR (400 MHz, CDCl₃): δ ppm 0.83 - 0.92 (m, 1 H) 0.95 (t, J=7.44 Hz, 3 H) 1.14 - 1.18 (m, 3 H) 1.29 (d, J=6.25 Hz, 3 H) 1.30 - 1.33 (m, 9 H) 1.33 - 1.50 (m, 3 H) 1.56 - 1.74 (m, 8 H) 1.75 - 2.07 (m, 5 H) 2.11 - 2.37 (m, 4 H) 2.41 (m, 1 H) 2.88 (m, 1 H) 2.99 - 3.06 (m, 1 H) 3.06 - 3.16 (m, 2 H) 3.24 - 3.33 (m, 1 H) 3.36 (s, 3 H) 3.41 (s, 3 H) 3.44 - 3.49 (m, 2 H) 3.50 (d, J=2.50 Hz, 7 H) 3.56 (s, 4 H) 3.72 (br d, J=9.63 Hz, 1 H) 4.28 - 4.43 (m, 3 H) 4.85 (d, J=1.25 Hz, 1 H) 4.87 - 4.94 (m, 1 H) 5.04 (br t, J=8.25 Hz, 1 H) 5.31 (m, 1 H) 5.64 - 5.82 (m, 4 H) 5.85 - 5.92 (m, 1 H) 6.79 (s, 1 H).
LCMS (ESI+): m/z 872.5 [M+H]⁺.

### Example 2: Synthesis of compound J1B

Butenyl spinosyn α1 (800 mg, 1.06 mmol) was dissolved in acetonitrile (16 mL) and isopropyl 2-bromoacetate (959.4 mg, 5.3 mmol) and 1,2,2,6,6-pentamethylpiperidine (327.77 mg, 2.11 mmol) were added. The solution was heated to 60 °C and stirred for 6 hours. The reaction mixture was filtered and crude product was purified by reversed phase preparative HPLC. Fractions containing pure product were combined, adjusted to pH 7 and lyophilized to give white solid. The solid was suspended in acetonitrile (10 mL) and filtered to remove ammonium chloride. The filtrate was lyophilized to give J1B (392 mg, 41.5% yield, 92.9% purity) as white solid. The structure of the compound was determined using LCMS and ¹H NMR.

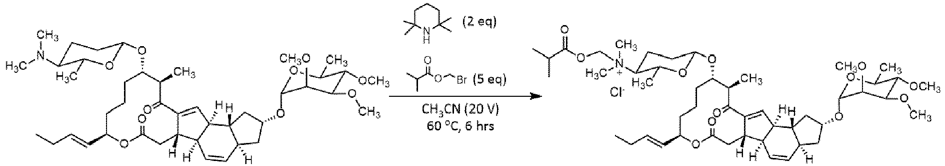

Analytical data for compound J1B:
¹H NMR (400 MHz, CDCl₃): δ ppm 0.82 - 0.91 (m, 1 H) 0.94 (t, J=7.44 Hz, 3 H) 1.15 (br d, J=6.75 Hz, 3 H) 1.20 - 1.30 (m, 10 H) 1.31- 1.50 (m, 3 H) 1.51- 1.80 (m, 8 H) 1.82 - 2.05 (m, 4 H) 2.09 - 2.33 (m, 4 H) 2.39 (m, 1 H) 2.76 (m, 1 H) 2.82 - 2.92 (m, 1 H) 2.97 - 3.16 (m, 3 H) 3.21 - 3.33 (m, 1 H) 3.38 - 3.52 (m, 15 H) 3.55 (s, 4 H) 3.70 (br d, J=9.51 Hz, 1 H) 4.16 (br s, 1 H) 4.26 - 4.42 (m, 2 H) 4.81 - 4.92 (m, 2 H) 4.98 - 5.07 (m, 1 H) 5.29 (m, 1 H) 5.63 - 5.92 (m, 5 H) 6.77 (s, 1 H).
LCMS (ESI+): m/z 858.5 [M+H]⁺.

### Example 3: Synthesis of compound J7

To a solution of Butenyl spinosyn α1 (800 mg, 1.06 mmol) in acetonitrile (16 mL) chloromethyl isopropyl carbonate (805.7 mg, 5.3 mmol), sodium iodide (791.01 mg, 5.3 mmol) and 1,2,2,6,6-pentamethylpiperidine (327.77 mg, 2.11 mmol) were added. The solution was heated to 60 °C and stirred for 6 hours. The reaction mixture was filtered and crude product was purified by reversed phase preparative HPLC. The elution solution was adjusted to pH 7 and lyophilized to give white solid. The solid was suspended in acetonitrile (10 mL) and filtered to remove ammonium chloride. The filtrate was lyophilized to give J7 (391 mg, 40.7% yield, 99.3% purity) as white solid. The correct structure of the compound was determined using 1H NMR.

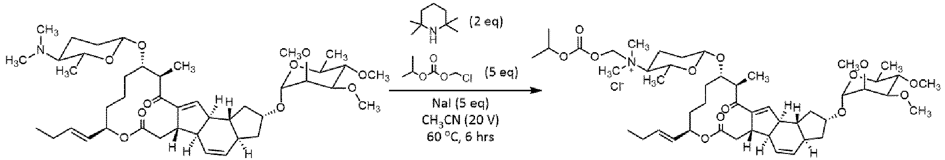

Analytical data for compound J7:
¹H NMR (400 MHz, CDCl₃): δ ppm 0.83 - 0.92 (m, 1 H) 0.95 (t, J=7.44 Hz, 3 H) 1.16 (d, J=6.75 Hz, 3 H) 1.25 (s, 1 H) 1.28 (d, J=6.25 Hz, 3 H) 1.33 (br d, J=6.13 Hz, 1 H) 1.37 (m, 6 H) 1.39 - 1.50 (m, 2 H) 1.57 - 1.81 (m, 9 H) 1.86 - 2.05 (m, 4 H) 2.10 - 2.34 (m, 4 H) 2.40 (m, 1 H) 2.87 (m, 1 H) 3.03 (br d, J=7.38 Hz, 1 H) 3.05 - 3.15 (m, 2 H) 3.22 - 3.34 (m, 1 H) 3.40 - 3.48 (m, 7 H) 3.49 (d, J=2.38 Hz, 7 H) 3.56 (s, 3 H) 3.71 (br d, J=9.51 Hz, 1 H) 4.17 (br t, J=7.25 Hz, 1 H) 4.27 - 4.45 (m, 2 H) 4.85 (d, J=1.38 Hz, 1 H) 4.87 - 4.93 (m, 1 H) 4.93 - 4.99 (m, 1 H) 4.99 - 5.07 (m, 1 H) 5.30 (m, 1 H) 5.64 - 5.80 (m, 4 H) 5.84 - 5.91 (m, 1 H) 6.78 (s, 1 H).
LCMS (ESI+): m/z 874.4 [M+H]⁺.

### Example 4: Stability of spinosyn compounds

Stability of compounds J1A, J1B and J7 was evaluated at different pH values. Differential stability / conversion to the active ingredient is expected to result in condition-specific activation and thereby to higher specificity towards target pests.

Stock solutions of the compounds were prepared at concentration of 0.1 mol/L. 10 µL of the stock solutions were transferred to 0.75 mL of the buffer solutions with pH values of 4, 5, 9 and 11. The resulting solutions were incubated for 10 minutes and 3 and 24 h. After specified times samples were analyzed using LC-MS. The compounds J1A, J1B, J7 and the active compound butenyl spinosyn were quantified. Results are presented in table 1. It is seen that the three compounds are converted to the active form preferably in alkaline conditions.

**Table 1: Stability of J1A, J1B and J7 at different pH values**

| **pH** | **Time [h]** | **J1A** | | **J1B** | | **J7** | |
|---|---|---|---|---|---|---|---|
| | | **BuSPN Area%** | **J1A Area%** | **BuSPN Area%** | **J1B Area%** | **BuSPN Area%** | **J7 Area%** |
| 4 | 0,16 | 0,0% | 5,7% | 3,1% | 5,2% | 0,0% | 19,2% |
| | 3 | 0,0% | 2,0% | 2,3% | 2,8% | 0,0% | 4,1% |
| | 24 | 0,0% | 0,1% | 2,7% | 2,0% | 0,0% | 3,8% |
| 5 | 0,16 | 0,1% | 99,4% | 35,4% | 60,4% | 0,2% | 99,2% |
| | 3 | 0,1% | 99,3% | 35,5% | 60,4% | 0,3% | 99,7% |
| | 24 | 1,7% | 97,4% | 37,4% | 58,2% | 0,5% | 99,2% |
| 9 | 0,16 | 32,6% | 66,4% | 72,6% | 21,1% | 44,4% | 54,2% |
| | 3 | 71,8% | 26,6% | 94,9% | 0,2% | 90,8% | 7,6% |
| | 24 | 46,6% | 0,0% | 85,9% | 0,5% | 93,8% | 0,6% |
| 11 | 0,16 | 80,7% | 18,4% | 88,0% | 1,8% | 93,1% | 6,7% |
| | 3 | 98,0% | 0,5% | 95,6% | 0,1% | 99,8% | 0,0% |
| | 24 | NA | NA | 91,5% | 0,5% | 99,4% | 0,0% |

It is known that lepidopteran larvae have high pH in their midgut. Therefore, the provided compounds may be found to be active specifically on lepidopteran larvae and not on numerous beneficial insects such as honey bees.

### Example 5: Evaluation of biological activity of spinosyn compounds

Insecticide activities of the compounds of this invention were assessed in comparison with commercially available samples of of spinosad (LGC (Dr. Ehrenstorfer); Product code: DRE-C16972830) and spinetoram (LGC (Dr. Ehrenstorfer); Product code: DRE-C16972770). Solutions of spinosyn compounds were prepared by adding 0.05% Tween 80, lowering pH to 5.95 and sonicating. Two concentrations of each compound were used, 0.1 µg/mL and 0.3 µg/mL, with each condition tested in four parallels.

Freshly collected corn leaves were dipped into the prepared solutions. After dipping leaves were left to dry at room temperature for 1 hour. Each dry leaf was then infested with 4 larvae of *Ostrinia nubilialis* (development stage L1), and the experimental device was incubated at 21 °C - 23°C for 24 h in order to optimize insect development. At three timings post infestation (1 day, 3 days and 6 days), the insecticide efficacy is evaluated from the number of living larvae using the Henderson-Tilton method. Untreated control was integrated for normalization and validation of larvae health.

**Table 2: Comparison of efficacy of tested spinosyn compounds**

| **Treatment** | **Dose (µg a.i./ml)** | % **Efficacy** | | |
|---|---|---|---|---|
| | | 1 **day** | **3 days** | 6 **days** |
| SPN | 0.1 | 20% | 31% | 60% |
| | 0.3 | 25% | 66% | 100% |
| Spinoteram | 0.1 | 27% | 60% | 85% |
| | 0.3 | 31% | 86% | 97% |
| J1A | 0.1 | 2% | 44% | 89% |
| | 0.3 | 0% | 63% | 100% |
| J1B | 0.1 | 35% | 78% | 91% |
| | 0.3 | 50% | 98% | 100% |
| J7 | 0.1 | 31% | 73% | 91% |
| | 0.3 | 44% | 91% | 100% |

These results show that the compounds J1A, J1B and J7 show extremely potent insecticidal activity on *Ostrinia nubilialis* larvae. The activity is comparable or superior to Spinosad and spinetoram.

### Example 6: Evaluation of kinetic solubility of spinosyn compounds

Solubility of the compounds of the invention was assessed using kinetic solubility assay and compared to solubility of butenyl spinosyn. Stock solutions of spinosyn compounds were prepared in DMSO at 10 mM concentration). 10 uL of the stock solutions were added into 490 of in K.S. 5.4 buffer of pH 5.4 (80mM phosphoric acid, acetic acid, boric acid buffer, adjusted the pH to pH 5.4 with 1 N HCl and 1N NaOH) or K.S. 7.4 buffer with pH of 7.4 (80mM phosphoric acid, acetic acid, boric acid buffer, adjusted the pH to pH 7.4 with 1 N HCl and 1N NaOH). The solutions were incubated with shaking at room temperature (25 ±2 °C) for 24 hours. 200 uL of each solution were then transferred into a new MultiScreen filter plate (Membrane of polycarbonate) and filtered by millipore vacuum manifold, and the filtrate was collected. The concentrtions of tested compounds in the filtered samples were analyzed by HPLC analysis calibrated by a standard curve generated by injection of 3 standard solutions of the relevant compound with concentrations of 1, 20, 200 µM. The results of the testing are presented in Table 3. The conditions of HPLC analysis are presented in Table 4.

**Table 3. Comparison of solubility of spinosyn compounds at pH 5.4 and 7.4.**

| **Compound** | **K.S 5.4 Dose Conc (µM)** | **Kinetic Solubility pH=5.4 (µM)** | **K.S 7.4 Dose Conc (µM)** | **Kinetic Solubility pH=7.4 (µM)** |
|---|---|---|---|---|
| **Butenyl spinosyn** | 200 | 183.17 | 200 | 3.52 |
| **J1A** | 200 | 189.20 | 200 | 142.51 |
| **J7** | 200 | 191.79 | 200 | 144.75 |

**Table 4. Conditions of the HPLC analysis for determination of kinetic solubility.**

| | | | |
|---|---|---|---|
| Instrument | Agilent 1200 | | |
| Detector | DAD | | |
| Mobile phase | A: Water containing 0.37‰ TFA | | |
| | B: Acetonitrile containing 0.19‰ TFA | | |
| Gradient | Time | B | Flow rate |
| | (min) | (%) | ( mL/min) |
| | 0.00 | 5 | 1.0 |
| | 2.00 | 90 | 1.0 |
| | 2.50 | 90 | 1.0 |
| | 3.01 | 5 | 1.0 |
| | 4.00 | 5 | 1.0 |
| Column | Xbridge C18 (2.1x50 mm, 5 µm) | | |
| Injection Volume | 20 µL | | |

These results show that the compounds of the invention are dramatically more soluble in the neutral pH values (7.4) compared to butenyl spinosyn, which represents a big advantage in preparation of formulations based on aqueous media.

### List of certain references cited in the description

Donald R. Hahn, Gary Gustafson, Clive Waldron, Brian Bullard, James D. Jackson and Jon Mitchell; "Butenyl-spinosyns, a natural example of genetic engineering of antibiotic biosynthetic genes"; J Ind Microbiol Biotechnol.; 33(2):94-104 (2006) https://doi.org/10.1007/s10295-005-0016-9
Chao Guo, Weiqun Guo, Yuchun Liu and Chao Wang; "Complete genome sequence of butenyl-spinosyn-producing Saccharopolyspora strain ASAGF58"; Annals of Microbiology; 70, 46 (2020) https://doi.org/10.1186/s13213-020-01587-4
Paul Lewer, Donald R. Hahn, Laura L. Karr, Dennis O. Duebelbeis, Jeffrey R. Gilbert, Gary D. Crouse, Thomas Worden, Thomas C. Sparks, Pat McKamey, Rex Edwards abd Paul R. Graupner; "Discovery of the butenyl-spinosyn insecticides: novel macrolides from the new bacterial strain Saccharopolyspora pogona"; Bioorg Med Chem.; 17(12):4185-96 (2009) https://doi.org/10.1016/j.bmc.2009.02.035
John Daeuble, Thomas C. Sparks, Peter Johnson and Paul R. Graupner; "Modification of the butenyl-spinosyns utilizing cross-metathesis"; Bioorg Med Chem.; 17(12):4197-205 (2009) https://doi.org/10.1016/j.bmc.2009.02.036Get
Herbert A Kirst; "The spinosyn family of insecticides: realizing the potential of natural products research"; J Antibiot; 63:101-111 (2010) https://doi.org/10.1038/ja.2010.5
Carl V. DeAmicis, James E. Dripps, Chris J. Hatton, and Laura L. Karr; "Physical and Biological Properties of the Spinosyns: Novel Macrolide Pest-Control Agents from Fermentation"; ACS Symposium Series; Vol. 658, Chapter 11: 144-154 (1997) https://doi.org/10.1021/bk-1997-0658.ch011
Da-You Ma, Long-Long Wang, Qin Lai, Kun-Jian Peng, Xuan Li, Zeng-Xia Li, Li-Jun Liu, Zhi-Yong Luo and Su-You Liu; "Synthesis and antiproliferative activities of novel quartenary ammonium spinosyn derivatives"; Bioorg Med Chem Lett.; 28(20):3346-3349 (2018) https://doi.org/10.1016/j.bmcl.2018.09.005
Raghavendra Ramachanderan and Bernd Schaefer; "Spinosyn insecticides"; ChemTexts; 6, 20 (2020) https://doi.org/10.1007/s40828-020-00113-y

## Claims

1. A compound of general formula (II) 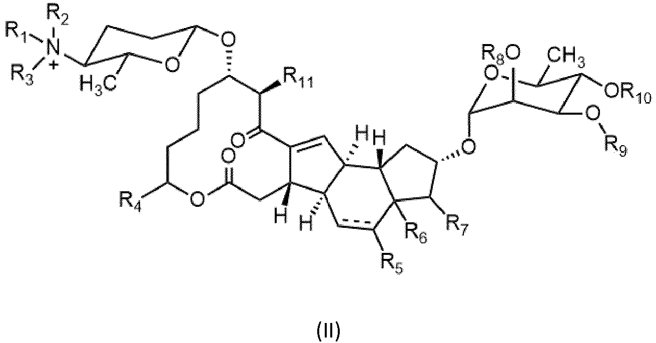 wherein
the dashed line is a single bond, a double bond or an epoxide;
R₁ is *-(CH₂)n-O-C(O)-R₁b, wherein
* denotes an attachment to the nitrogen atom;
n is an integer ranging from 1 to 10, or is an integer ranging from 1 to 5; and
R₁b is selected from the group consisting of *-C(R₂b)₃, *-N(R₂b)₂, *-OH and *-OC(R₂b)₃, wherein
* denotes an attachment to the carbonyl carbon atom; and
each R₂b is independently selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₂ is selected from the group consisting of substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₃ is selected from the group consisting of substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₄ is selected from the group consisting of unsubsituted ethyl, unsubsituted propyl, unsubsituted butyl, unsubsituted 1-butenyl, unsubsituted 1,3-butadienyl and unsubsituted 3-hydroxy-1-butenyl, preferably is unsubsituted ethyl or unsubsituted 1-butenyl, more preferably is unsubsituted 1-butenyl;
R₅ is -H or unsubsituted methyl; preferably is -H;
R₆ is -H;
R₇ is -H or -OR_{7'}, wherein R_{7'} is hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₈ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl;
R₉ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl,
substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl;
R₁₀ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is optionally substituted C₁-C₂ alkyl; and
R₁₁ is selected from the group consisting of hydrogen and substituted or unsubsituted C₁-C₂ alkyl; preferably is hydrogen or substituted or unsubstituted methyl; more preferably is hydrogen or unsubstituted methyl; most preferably is unsubstituted methyl,
in the form of a corresponding salt thereof.

2. The compound according to claim 1, wherein the compound is a compound of general formula (III) 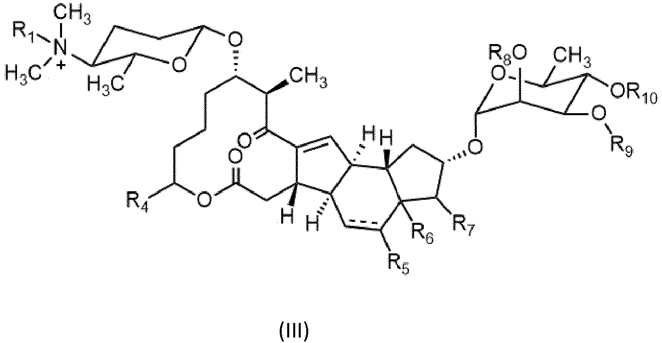 wherein
the dashed line is a single bond, a double bond or an epoxide;
R₁ is *-(CH₂)n-O-C(O)-R₁b, wherein
* denotes an attachment to the nitrogen atom;
n is an integer ranging from 1 to 10, or is an integer ranging from 1 to 5; and
R₁b is selected from the group consisting of *-C(R₂b)₃, *-N(R₂b)₂, *-OH and *-OC(R₂b)₃, wherein
* denotes an attachment to the carbonyl carbon atom; and
each R₂b is independently selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₄ is selected from the group consisting of unsubsituted ethyl, unsubsituted propyl, unsubsituted butyl, unsubsituted 1-butenyl, unsubsituted 1,3-butadienyl and unsubsituted 3-hydroxy-1-butenyl, preferably is unsubsituted ethyl or unsubsituted 1-butenyl, more preferably is unsubsituted 1-butenyl;
R₅ is -H or unsubsituted methyl; preferably is -H;
R₆ is -H;
R₇ is -H or -OR_{7'}, wherein R_{7'} is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted;
R₈ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl;
R₉ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl; and
R₁₀ is selected from the group consisting of hydrogen, substituted or unsubsituted C₁-C₂₀ alkyl, substituted or unsubsituted C₂-C₂₀ alkenyl, substituted or unsubsituted C₂-C₂₀ alkynyl, substituted or unsubsituted C₃-C₁₂ cycloalkyl, substituted or unsubsituted C₃-C₁₂ aryl, substituted or unsubsituted C₃-C₁₂ heterocyclyl, -(C₁-C₂₀ alkyl)-(C₃-C₁₂ cycloalkyl) with the cycloalkyl being optionally substituted, -(C₁-C₂₀ alkyl)-(C₃-C₁₂ aryl) with the aryl being optionally substituted, and -(C₁-C₂₀ alkyl)-(C₃-C₁₂ heterocyclyl) with the heterocyclyl being optionally substituted; preferably is substituted or unsubsituted C₁-C₂ alkyl,
in the form of a corresponding salt thereof.

3. The compound according to claim 1 or 2, wherein R₄ is unsubstituted ethyl or unsubstituted 1-butenyl and/or R₅ is -H and/or R₇ is -H.

4. The compound according to any one of claims 1 to 3, wherein each of R₈, R₉ and R₁₀ is substituted or unsubsituted C₁-C₆ alkyl.

5. The compound according to any one of claims 1 to 4, wherein each of R₈, R₉ and R₁₀ is -CH₃.

6. The compound according to any one of claims 1 to 5, wherein n is 1, 2, 3, 4 or 5.

7. The compound according to any one of claims 1 to 6, wherein R₁b is *-C(R₂b)₃ or *-OC(R₂b)₃.

8. The compound according to any one of claims 1 to 7, wherein each R₂b is independently selected from the group consisting of hydrogen, substituted or unsubsituted C₁-C₆ alkyl, substituted or unsubsituted C₂-C₆ alkenyl, substituted or unsubsituted C₂-C₆ alkynyl, substituted or unsubsituted C₃-C₁₂ cycloalkyl, substituted or unsubsituted C₆-C₁₀ aryl, substituted or unsubsituted C₅-C₁₀ heterocyclyl, -(C₁-C₆ alkyl)-(C₃-C₁₂ cycloalkyl) with the cycloalkyl being optionally substituted, -(C₁-C₆ alkyl)-(C₆-C₁₀ aryl) with the aryl being optionally substituted, and -(C₁-C₆ alkyl)-(C₅-C₁₀ heterocyclyl) with the heterocyclyl being optionally substituted.

9. The compound according to any one of claims 1 to 8, wherein R₂ is substituted or unsubsituted C₁-C₆ alkyl and/or R₃ is substituted or unsubsituted C₁-C₆ alkyl.

10. The compound according to any one of claims 1 to 2, which is selected from the group consisting of 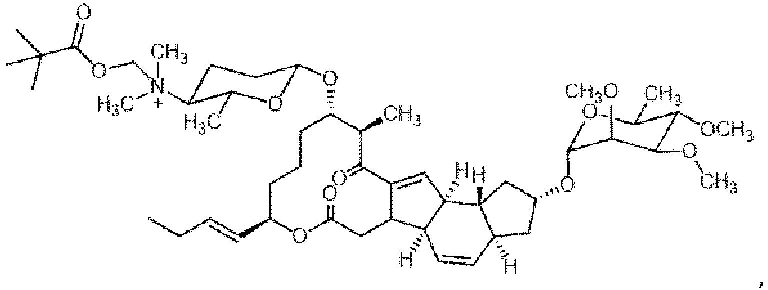 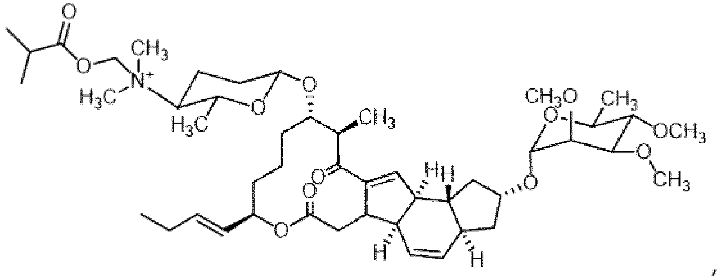 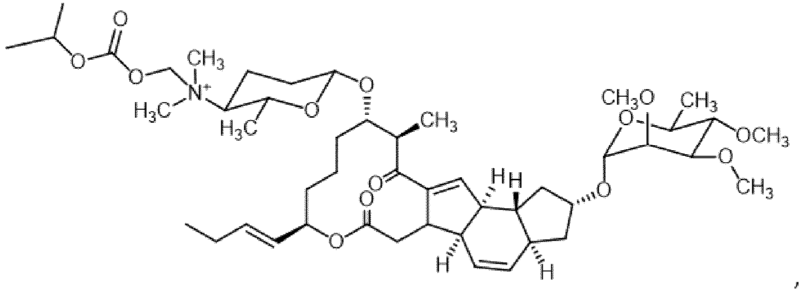 in the form of a corresponding salt thereof.

11. The compound according to any one of claims 1-10, wherein said corresponding salt is with an anion selected from the group consisting of chloride, sulfate, nitrate, phosphate, citrate, tartrate, acetate, lactate, propionate and gluconate.

12. Composition comprising a compound according to any one of claims 1 to 11 and one or more physiologically acceptable adjuvants.

13. Use of a compound according to any one of claims 1 to 11 or a composition according to claim 12 as a pesticide.

14. A non-therapeutical method for controlling a pest, such as a plant pest, comprises contacting a pest, such as a plant pest, with a compound according to any one of claims 1 to 11 or a composition according to claim 12.

15. A method for protecting a plant against a plant pest, comprising the step of: applying a compound according to any one of claims 1 to 11 or a composition according to claim 12 to a plant in need thereof.

## Patentansprüche

1. Verbindung einer allgemeinen Formel (II) 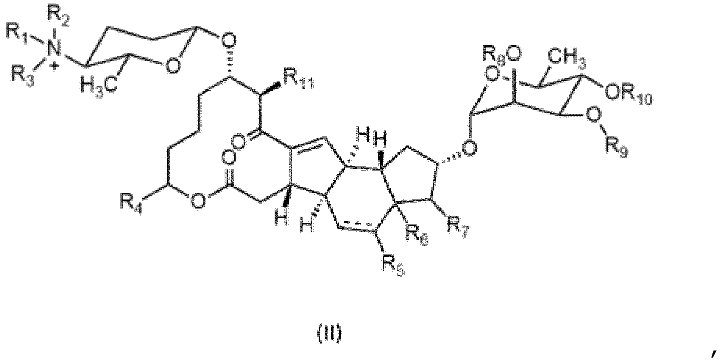 wobei
die gestrichelte Linie eine Einfachbindung, eine Doppelbindung oder ein Epoxid ist;
R₁ *-(CH₂)n-O-C(O)-R₁b ist, wobei
* eine Anheftung an dem Stickstoffatom bezeichnet;
n eine Ganzzahl im Bereich von 1 bis 10 ist oder eine Ganzzahl im Bereich von 1 bis 5 ist; und
R₁b aus der Gruppe ausgewählt ist, die aus *-C(R₂b)₃, *-N(R₂b)₂, *-OH und *-OC(R₂b)₃ besteht, wobei
* eine Anheftung an dem Carbonylkohlenstoffatom bezeichnet; und
jedes R₂b unabhängig aus der Gruppe ausgewählt ist, die aus Folgendem besteht: Wasserstoff, substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, - (C₁-C₂₀-Alkyl)- (C₃-C₁₂-Cycloalkyl), wobei das Cycloalkyl gegebenenfalls substituiert ist, -(C₁-C₂₀-Alkyl)-(C₃-C₁₂-Aryl), wobei das Aryl gegebenenfalls substituiert ist, und -(C₁-C₂₀-Alkyl)-(C₃-C₁₂-Heterocyclyl), wobei das Heterocyclyl gegebenenfalls substituiert ist;
R₂ aus der Gruppe ausgewählt ist, die aus Folgendem besteht: substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, - (C₁-C₂₀-Alkyl)-(C₃-C₁₂-Cycloalkyl), wobei das Cycloalkyl gegebenenfalls substituiert ist, -(C₁-C₂₀-Alkyl)-(C₃-C₁₂-Aryl), wobei das Aryl gegebenenfalls substituiert ist, und -(C₁-C₂₀-Alkyl)-(C₃-C₁₂-Heterocyclyl), wobei das Heterocyclyl gegebenenfalls substituiert ist;
R₃ aus der Gruppe ausgewählt ist, die aus Folgendem besteht: substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, - (C₁-C₂₀-Alkyl)-(C₃-C₁₂-Cycloalkyl), wobei das Cycloalkyl gegebenenfalls substituiert ist, -(C₁-C₂₀-Alkyl)-(C₃-C₁₂-Aryl), wobei das Aryl gegebenenfalls substituiert ist, und -(C₁-C₂₀-Alkyl)-(C₃-C₁₂-Heterocyclyl), wobei das Heterocyclyl gegebenenfalls substituiert ist;
R₄ aus der Gruppe ausgewählt ist, die aus nicht substituiertem Ethyl, nicht substituiertem Propyl, nicht substituiertem Butyl, nicht substituiertem 1-Butenyl, nicht substituiertem 1,3-Butadienyl und nicht substituiertem **3-**Hydroxy-1-butenyl besteht, bevorzugt nicht substituiertes Ethyl oder nicht substituiertes 1-Butenyl ist, bevorzugter nicht substituiertes 1-Butenyl ist;
R₅ -H oder nicht substituiertes Methyl ist; bevorzugt -H ist;
R₆ -H ist;
R₇ -H oder -OR₇, ist, wobei R₇, Folgendes ist: Wasserstoff, substituiertes oder nicht substituiertes C₁-C₂₀-Alkyl, substituiertes oder nicht substituiertes C₂-C₂₀-Alkenyl, substituiertes oder nicht substituiertes C₂-C₂₀-Alkinyl, substituiertes oder nicht substituiertes C₃-C₁₂-Cycloalkyl, substituiertes oder nicht substituiertes C₃-C₁₂-Aryl, substituiertes oder nicht substituiertes C₃-C₁₂-Heterocyclyl, - (C₁-C₂₀-Alkyl)-(C₃-C₁₂-Cycloalkyl), wobei das Cycloalkyl gegebenenfalls substituiert ist, -(C₁-C₂₀-Alkyl)-(C₃-C₁₂-Aryl), wobei das Aryl gegebenenfalls substituiert ist, und -(C₁-C₂₀-Alkyl)-(C₃-C₁₂-Heterocyclyl), wobei das Heterocyclyl gegebenenfalls substituiert ist;
R₈ aus der Gruppe ausgewählt ist, die aus Folgendem besteht: Wasserstoff, substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, - (C₁-C₂₀-Alkyl)-(C₃-C₁₂-Cycloalkyl), wobei das Cycloalkyl gegebenenfalls substituiert ist, -(C₁-C₂₀-Alkyl)-(C₃-C₁₂-Aryl), wobei das Aryl gegebenenfalls substituiert ist, und -(C₁-C₂₀-Alkyl)-(C₃-C₁₂-Heterocyclyl), wobei das Heterocyclyl gegebenenfalls substituiert ist; bevorzugt substituiertes oder nicht substituiertes C₁-C₂-Alkyl ist;
R₉ aus der Gruppe ausgewählt ist, die aus Folgendem besteht: Wasserstoff, substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, - (C₁-C₂₀-Alkyl)-(C₃-C₁₂-Cycloalkyl), wobei das Cycloalkyl gegebenenfalls substituiert ist, -(C₁-C₂₀-Alkyl)-(C₃-C₁₂-Aryl), wobei das Aryl gegebenenfalls substituiert ist, und -(C₁-C₂₀-Alkyl)-(C₃-C₁₂-Heterocyclyl), wobei das Heterocyclyl gegebenenfalls substituiert ist; bevorzugt gegebenenfalls substituiertes C₁-C₂-Alkyl ist;
R₁₀ aus der Gruppe ausgewählt ist, die aus Folgendem besteht: Wasserstoff, substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, -(C₁-C₂₀-Alkyl)-(C₃-C₁₂-Cycloalkyl), wobei das Cycloalkyl gegebenenfalls substituiert ist, -(C₁-C₂-Alkyl)-(C₃-C₁₂-Aryl), wobei das Aryl gegebenenfalls substituiert ist, und - (C₁-C₂₀-Alkyl)-(C₃-C₁₂-Heterocyclyl), wobei das Heterocyclyl gegebenenfalls substituiert ist; bevorzugt gegebenenfalls substituiertes C₁-C₂-Alkyl ist; und
R₁₁ aus der Gruppe ausgewählt ist, die aus Wasserstoff und substituiertem oder nicht substituiertem C₁-C₂-Alkyl besteht; bevorzugt Wasserstoff oder substituiertes oder nicht substituiertes Methyl ist; bevorzugter Wasserstoff oder nicht substituiertes Methyl ist; am bevorzugtesten nicht substituiertes Methyl ist,
in der Form eines entsprechenden Salzes davon.

2. Verbindung nach Anspruch 1, wobei die Verbindung eine Verbindung einer allgemeinen Formel (III) ist 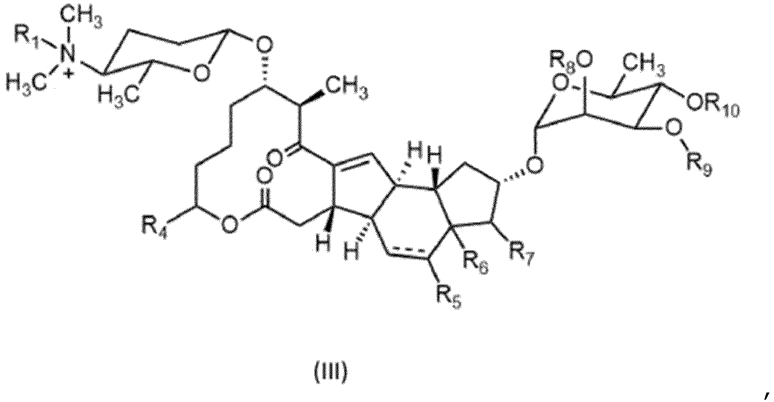 wobei
die gestrichelte Linie eine Einfachbindung, eine Doppelbindung oder ein Epoxid ist;
R₁*-(CH₂)n-O-C(O)-R₁b ist, wobei
* eine Anheftung an dem Stickstoffatom bezeichnet;
n eine Ganzzahl im Bereich von 1 bis 10 ist oder eine Ganzzahl im Bereich von 1 bis 5 ist; und
R₁b aus der Gruppe ausgewählt ist, die aus *-C(R₂b)₃, *-N(R₂b)₂, *-OH und *-OC(R₂b)₃ besteht, wobei
* eine Anheftung an dem Carbonylkohlenstoffatom bezeichnet; und
jedes R₂b unabhängig aus der Gruppe ausgewählt ist, die aus Folgendem besteht: Wasserstoff, substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, - (C₁-C₂₀-Alkyl)- (C₃-C₁₂-Cycloalkyl), wobei das Cycloalkyl gegebenenfalls substituiert ist, -(C₁-C₂₀-Alkyl)-(C₃-C₁₂-Aryl), wobei das Aryl gegebenenfalls substituiert ist, und - (C₁-C₂₀-Alkyl)-(C₃-C₁₂-Heterocyclyl), wobei das Heterocyclyl gegebenenfalls substituiert ist;
R₄ aus der Gruppe ausgewählt ist, die aus nicht substituiertem Ethyl, nicht substituiertem Propyl, nicht substituiertem Butyl, nicht substituiertem 1-Butenyl, nicht substituiertem 1,3-Butadienyl und nicht substituiertem **3-**Hydroxy-1-butenyl besteht, bevorzugt nicht substituiertes Ethyl oder nicht substituiertes 1-Butenyl ist, bevorzugter nicht substituiertes 1-Butenyl ist;
R₅ -H oder nicht substituiertes Methyl ist; bevorzugt **-H** ist;
R₆ -H ist;
R₇ -H oder -OR₇, ist, wobei R₇, aus der Gruppe ausgewählt ist, die aus Folgendem besteht: Wasserstoff, substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, - (C₁-C₂₀-Alkyl)-(C₃-C₁₂-Cycloalkyl), wobei das Cycloalkyl gegebenenfalls substituiert ist, -(C₁-C₂₀-Alkyl)-(₃-C₁₂-Aryl), wobei das Aryl gegebenenfalls substituiert ist, und -(C₁-C₂₀-Alkyl)-(C₃-C₁₂-Heterocyclyl), wobei das Heterocyclyl gegebenenfalls substituiert ist;
R₈ aus der Gruppe ausgewählt ist, die aus Folgendem besteht: Wasserstoff, substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, - (C₁-C₂₀-Alkyl)-(C₃-C₁₂-Cycloalkyl), wobei das Cycloalkyl gegebenenfalls substituiert ist, -(C₁-C₂₀-Alkyl)-(C₃-C₁₂-Aryl), wobei das Aryl gegebenenfalls substituiert ist, und -(C₁-C₂₀-Alkyl)-(C₃-C₁₂-Heterocyclyl), wobei das Heterocyclyl gegebenenfalls substituiert ist; bevorzugt substituiertes oder nicht substituiertes C₁-C₂-Alkyl ist;
R₉ aus der Gruppe ausgewählt ist, die aus Folgendem besteht: Wasserstoff, substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, - (C₁-C₂₀-Alkyl)-(C₃-C₁₂-Cycloalkyl), wobei das Cycloalkyl gegebenenfalls substituiert ist, -(C₁-C₂₀-Alkyl)-(C₃-C₁₂-Aryl), wobei das Aryl gegebenenfalls substituiert ist, und -(C₁-C₂₀-Alkyl)-(C₃-C₁₂-Heterocyclyl), wobei das Heterocyclyl gegebenenfalls substituiert ist; bevorzugt substituiertes oder nicht substituiertes C₁-C₂-Alkyl ist; und
R₁₀ aus der Gruppe ausgewählt ist, die aus Folgendem besteht: Wasserstoff, substituiertem oder nicht substituiertem C₁-C₂₀-Alkyl, substituiertem oder nicht substituiertem C₂-C₂₀-Alkenyl, substituiertem oder nicht substituiertem C₂-C₂₀-Alkinyl, substituiertem oder nicht substituiertem C₃-C₁₂-Cycloalkyl, substituiertem oder nicht substituiertem C₃-C₁₂-Aryl, substituiertem oder nicht substituiertem C₃-C₁₂-Heterocyclyl, -(C₁-C₂₀-Alkyl)-(C₃-C₁₂-Cycloalkyl), wobei das Cycloalkyl gegebenenfalls substituiert ist, -(C₁-C₂₀-Alkyl)-(C₃-C₁₂-Aryl), wobei das Aryl gegebenenfalls substituiert ist, und - (C₁-C₂₀-Alkyl)-(C₃-C₁₂-Heterocyclyl), wobei das Heterocyclyl gegebenenfalls substituiert ist; bevorzugt substituiertes oder nicht substituiertes C₁-C₂-Alkyl ist,
in der Form eines entsprechenden Salzes davon.

3. Verbindung nach Anspruch 1 oder 2, wobei R₄ substituiertes Ethyl oder nicht substituiertes 1-Butenyl ist und/oder R₅ -H ist und/oder R₇ -H ist.

4. Verbindung nach einem der Ansprüche 1 bis 3, wobei jedes von R₈, R₉ und R₁₀ substituiertes oder nicht substituiertes C₁-C₆-Alkyl ist.

5. Verbindung nach einem der Ansprüche 1 bis 4, wobei jedes von R₈, R₉ und R₁₀ -CH₃ ist.

6. Verbindung nach einem der Ansprüche 1 bis 5, wobei n 1, 2, 3, 4 oder 5 ist.

7. Verbindung nach einem der Ansprüche 1 bis 6, wobei R₁b *-C(R₂b)₃ oder *-OC(R₂b)₃ ist.

8. Verbindung nach einem der Ansprüche 1 bis 7, wobei jedes R₂b unabhängig aus der Gruppe ausgewählt ist, die aus Folgendem besteht: Wasserstoff, substituiertem oder nicht substituiertem C₁-C₆-Alkyl, substituiertem oder nicht substituiertem C₂-C₆-Alkenyl, substituiertem oder nicht substituiertem C₂-C₆-Alkinyl, substituiertem oder nicht substituiertem C₃-C₁₂-Cycloalkyl, substituiertem oder nicht substituiertem C₆-C₁₀-Aryl, substituiertem oder nicht substituiertem C₅-C₁₀-Heterocyclyl, - (C₁-C₆-Alkyl)-(C₃-C₁₂-Cycloalkyl), wobei das Cycloalkyl gegebenenfalls substituiert ist, -(C₁-C₆-Alkyl)-(C₆-C₁₀-Aryl), wobei das Aryl gegebenenfalls substituiert ist, und -(C₁-C₆-Alkyl)-(C₅-C₁₀-Heterocyclyl), wobei das Heterocyclyl gegebenenfalls substituiert ist.

9. Verbindung nach einem der Ansprüche 1 bis 8, wobei R₂ substituiertes oder nicht substituiertes C₁-C₆-Alkyl ist und/oder R₃ substituiertes oder nicht substituiertes C₁-C₆-Alkyl ist.

10. Verbindung nach einem der Ansprüche 1 bis 2, die aus der Gruppe ausgewählt ist, die aus Folgendem besteht: 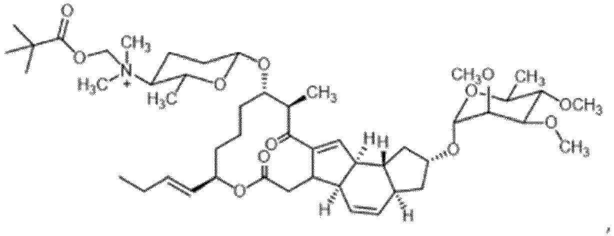 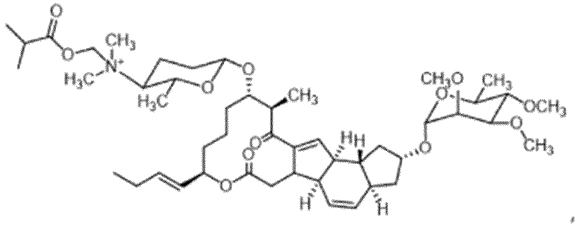 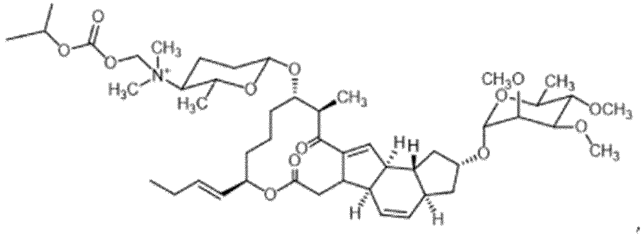 in der Form eines entsprechenden Salzes davon.

11. Verbindung nach einem der Ansprüche 1-10, wobei das entsprechende Salz ein Anion aufweist, das aus der Gruppe ausgewählt ist, die aus Chlorid, Sulfat, Nitrat, Phosphat, Citrat, Tartrat, Acetat, Laktat, Propionat und Gluconat besteht.

12. Zusammensetzung, umfassend eine Verbindung nach einem der Ansprüche 1 bis 11 und ein oder mehrere physiologisch verträgliche Adjuvanzien.

13. Verwendung einer Zusammensetzung nach einem der Ansprüche 1 bis 11 oder einer Zusammensetzung nach Anspruch 12 als ein Pestizid.

14. Nicht therapeutisches Verfahren zum Bekämpfen eines Schädlings, wie etwa eines Pflanzenschädlings, umfassend Kontaktieren eines Schädlings, wie etwa eines Pflanzenschädlings, mit einer Zusammensetzung nach einem der Ansprüche 1 bis 11 oder einer Zusammensetzung nach Anspruch 12.

15. Verfahren zum Schützen einer Pflanze vor einem Pflanzenschädling, umfassend den folgenden Schritt: Anbringen einer Verbindung nach einem der Ansprüche 1 bis 11 oder einer Zusammensetzung nach Anspruch 12 auf eine dessen bedürftige Pflanze.

## Revendications

1. Composé de formule générale (II) 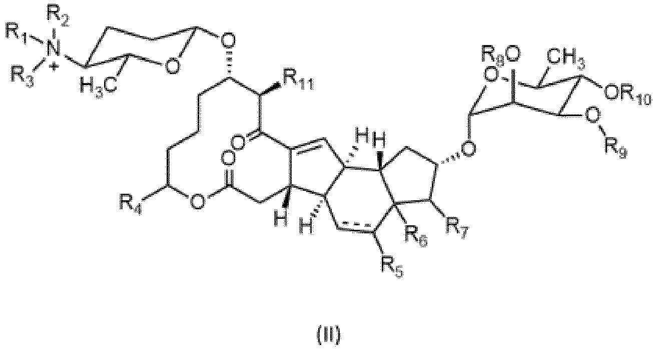 dans lequel
la ligne pointillée est une liaison simple, une double liaison ou un époxyde ;
R₁ est *-(CH₂)nOC(O)-R₁b, dans lequel
*désigne une fixation à l'atome d'azote ;
n est un entier compris entre 1 et 10, ou est un entier compris entre 1 et 5 ; et
R₁b est choisi dans le groupe constitué de *-C(R₂b)₃, *-N(R₂b)₂, *-OH et *-OC(R₂b)₃, dans lequel
* désigne une liaison à l'atome de carbone du carbonyle ; et
chaque R₂b est indépendamment sélectionné dans le groupe constitué par hydrogène, alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, - (alkyle C₁-C₂₀)-(cycloalkyle C₃-C₁₂) avec le cycloalkyle étant éventuellement substitué, -(alkyle C₁-C₂₀)- (aryle C₃-C₁₂) avec l'aryle étant éventuellement substitué, et -(alkyle C₁-C₂₀)-(hétérocyclyle C₃-C₁₂) avec l'hétérocyclyle étant éventuellement substitué ;
R₂ est choisi dans le groupe constitué par alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, -(alkyle C₁-C₂₀)- (cycloalkyle C₃-C₁₂) avec le cycloalkyle étant éventuellement substitué, -(alkyle C₁-C₂₀)-(aryle C₃-C₁₂) avec l'aryle étant éventuellement substitué, et - (alkyle C₁-C₂₀)- (hétérocyclyle C₃-C₁₂) avec l'hétérocyclyle étant éventuellement substitué ;
R₃ est choisi dans le groupe constitué par alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, - (alkyle C₁-C₂₀)- (cycloalkyle C₃-C₁₂) avec le cycloalkyle étant éventuellement substitué, -(alkyle C₁-C₂₀)-(aryle C₃-C₁₂) avec l'aryle étant éventuellement substitué, et -(alkyle C₁-C₂₀)- (hétérocyclyle C₃-C₁₂) avec l'hétérocyclyle étant éventuellement substitué ;
R₄ est choisi dans le groupe constitué par éthyle non substitué, propyle non substitué, butyle non substitué, 1-butényle non substitué, 1,3-butadiényle non substitué et 3-hydroxy-1-butényle non substitué, préférablement éthyle non substitué ou 1-butényle non substitué, plus préférablement 1-butényle non substitué ;
R₅ est -H ou méthyle non substitué ; préférablement -H ;
R₆ est -H ;
R₇ est -H ou -OR_{7'}, où R₇, est hydrogène, alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, -(alkyle C₁-C₂₀)- (cycloalkyle C₃-C₁₂) avec le cycloalkyle étant éventuellement substitué, -(alkyle C₁-C₂₀)-(aryle C₃-C₁₂) avec l'aryle étant éventuellement substitué, et -(alkyle C₁-C₂₀)-( hétérocyclyle C₃-C₁₂) avec l'hétérocyclyle étant éventuellement substitué ;
R₈ est choisi dans le groupe constitué par hydrogène, alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, -(alkyle C₁-C₂₀)-(cycloalkyle C₃-C₁₂) avec le cycloalkyle étant éventuellement substitué, - (alkyle C₁-C₂₀)-(aryle C₃-C₁₂) avec l'aryle étant éventuellement substitué, et -(alkyle C₁-C₂₀)- (hétérocyclyle C₃-C₁₂) avec l'hétérocyclyle étant éventuellement substitué ; préférablement alkyle C₁-C₂ substitué ou non substitué ;
R₉ est choisi dans le groupe constitué par hydrogène, alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, -(alkyle C₁-C₂₀)-(cycloalkyle C₃-C₁₂) avec le cycloalkyle étant éventuellement substitué, - (alkyle C₁-C₂₀)-(aryle C₃-C₁₂) avec l'aryle étant éventuellement substitué, et -(alkyle en C₁-C₂₀)-(hétérocyclyle C₃-C₁₂) avec l'hétérocyclyle étant éventuellement substitué ; préférablement alkyle C₁-C₂ éventuellement substitué ;
R₁₀ est choisi dans le groupe constitué par hydrogène, alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, -(alkyle C₁-C₂₀)-(cycloalkyle C₃-C₁₂) avec le cycloalkyle étant éventuellement substitué, -(alkyle C₁-C₂₀)-(aryle C₃-C₁₂) avec l'aryle étant éventuellement substitué, et - (alkyle C₁-C₂₀)- (hétérocyclyle C₃-C₁₂) avec l'hétérocyclyle étant éventuellement substitué ; préférablement alkyle C₁-C₂ éventuellement substitué ; et
R₁₁ est choisi dans le groupe constitué par hydrogène et alkyle C₁-C₂ substitué ou non substitué ; préférablement hydrogène ou méthyle substitué ou non substitué ; plus préférablement hydrogène ou méthyle non substitué ; le plus préférablement méthyle non substitué,
sous la forme d'un sel correspondant de celui-ci.

2. Composé selon la revendication 1, dans lequel le composé est un composé de formule générale (III). 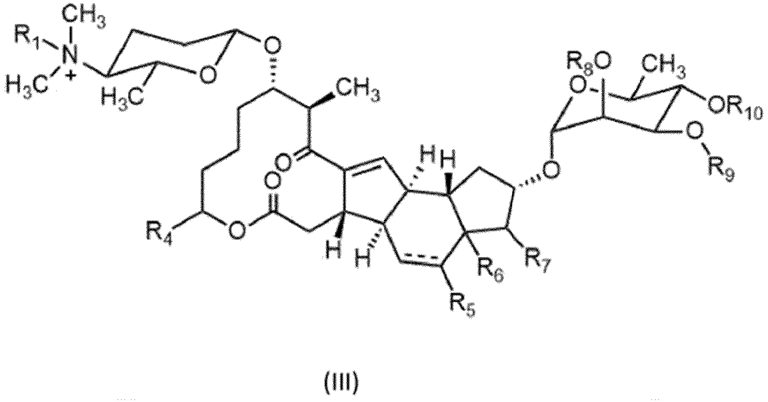 dans lequel
la ligne pointillée est une liaison simple, une double liaison ou un époxyde ;
R₁ est *-(CH₂)nOC(O)-R₁b, dans lequel
*désigne une fixation à l'atome d'azote ;
n est un entier compris entre 1 et 10, ou est un entier compris entre 1 et 5 ; et
R₁b est choisi dans le groupe constitué de *-C(R₂b)₃, *-N(R₂b)₂, *-OH et *-OC(R₂b)₃, dans lequel
* désigne une liaison à l'atome de carbone du carbonyle ; et
chaque R₂b est indépendamment sélectionné dans le groupe constitué par hydrogène, alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, - (alkyle C₁-C₂₀)- (cycloalkyle C₃-C₁₂) avec le cycloalkyle étant éventuellement substitué, - (alkyle C₁-C₂₀)- (aryle C₃-C₁₂) avec l'aryle étant éventuellement substitué, et -(alkyle C₁-C₂₀)-(hétérocyclyle C₃-C₁₂) avec l'hétérocyclyle étant éventuellement substitué ;
R₄ est choisi dans le groupe constitué par éthyle non substitué, propyle non substitué, butyle non substitué, 1-butényle non substitué, 1,3-butadiényle non substitué et 3-hydroxy-1-butényle non substitué, préférablement éthyle non substitué ou 1-butényle non substitué, plus préférablement 1-butényle non substitué ;
R₅ est -H ou méthyle non substitué ; préférablement -H ;
R₆ est -H ;
R₇ est -H ou -OR_{7'}, où R₇, est hydrogène, alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, -(alkyle C₁-C₂₀)- (cycloalkyle C₃-C₁₂) avec le cycloalkyle étant éventuellement substitué, -(alkyle C₁-C₂₀)-(aryle C₃-C₁₂) avec l'aryle étant éventuellement substitué, et -(alkyle C₁-C₂₀)-( hétérocyclyle C₃-C₁₂) avec l'hétérocyclyle étant éventuellement substitué ;
R₈ est choisi dans le groupe constitué par hydrogène, alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, -(alkyle C₁-C₂₀)-(cycloalkyle C₃-C₁₂) avec le cycloalkyle étant éventuellement substitué, - (alkyle C₁-C₂₀)-(aryle C₃-C₁₂) avec l'aryle étant éventuellement substitué, et -(alkyle C₁-C₂₀)- (hétérocyclyle C₃-C₁₂) avec l'hétérocyclyle étant éventuellement substitué ; préférablement alkyle C₁-C₂ substitué ou non substitué ;
R₉ est choisi dans le groupe constitué par hydrogène, alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, -(alkyle C₁-C₂₀)-(cycloalkyle C₃-C₁₂) avec le cycloalkyle étant éventuellement substitué, - (alkyle C₁-C₂₀)-(aryle C₃-C₁₂) avec l'aryle étant éventuellement substitué, et -(alkyle en C₁-C₂₀)-(hétérocyclyle C₃-C₁₂) avec l'hétérocyclyle étant éventuellement substitué ; préférablement alkyle C₁-C₂ éventuellement substitué ;
R₁₀ est choisi dans le groupe constitué par hydrogène, alkyle C₁-C₂₀ substitué ou non substitué, alcényle C₂-C₂₀ substitué ou non substitué, alcynyle C₂-C₂₀ substitué ou non substitué, cycloalkyle C₃-C₁₂ substitué ou non substitué, aryle C₃-C₁₂ substitué ou non substitué, hétérocyclyle C₃-C₁₂ substitué ou non substitué, -(alkyle C₁-C₂₀)-(cycloalkyle C₃-C₁₂) avec le cycloalkyle étant éventuellement substitué, -(alkyle C₁-C₂₀)-(aryle C₃-C₁₂) avec l'aryle étant éventuellement substitué, et -(alkyle C₁-C₂₀)- (hétérocyclyle C₃-C₁₂) avec l'hétérocyclyle étant éventuellement substitué ; préférablement alkyle C₁-C₂ éventuellement substitué,
sous la forme d'un sel correspondant de celui-ci.

3. Composé selon la revendication 1 ou 2, dans lequel R₄ est éthyle non substitué ou 1-butényle non substitué et/ou R₅ est - H et/ou R₇ est -H.

4. Composé selon l'une quelconque des revendications 1 à 3, dans lequel chacun de R₈, R₉ et R₁₀ est alkyle C₁-C₆ substitué ou non substitué.

5. Composé selon l'une quelconque des revendications 1 à 4, dans lequel chacun de R₈, R₉ et R₁₀ est -CH₃.

6. Composé selon l'une quelconque des revendications 1 à 5, dans lequel n est 1, 2, 3, 4 ou 5.

7. Composé selon l'une quelconque des revendications 1 à 6, dans lequel R₁b est *-C (R₂b)₃ ou *-OC(R₂b)₃.

8. Composé selon l'une quelconque des revendications 1 à 7, dans lequel chaque R₂b est indépendamment choisi dans le groupe constitué par hydrogène, alkyle C₁-C₆ substitué ou non substitué, alcényle C₂-C₆ substitué ou non substitué, alcynyle C₂-C₆ substitué ou non substitué, cycloalkyle C₃-C₁₂ substitué ou non substitué, aryle C₆-C₁₀ substitué ou non substitué, hétérocyclyle C₅-C₁₀ substitué ou non substitué, -(alkyle C₁-C₆)- (cycloalkyle C₃-C₁₂) avec le cycloalkyle étant éventuellement substitué, - (alkyle C₁-C₆)- (aryle C₆-C₁₀) avec l'aryle étant éventuellement substitué, et -(alkyle C₁-C₆)-(hétérocyclyle C₅-C₁₀) avec l'hétérocyclyle étant éventuellement substitué.

9. Composé selon l'une quelconque des revendications 1 à 8, dans lequel R₂ est alkyle C₁-C₆ substitué ou non substitué et/ou R₃ est alkyle C₁-C₆ substitué ou non substitué.

10. Composé selon l'une quelconque des revendications 1 à 2, choisi dans le groupe constitué par 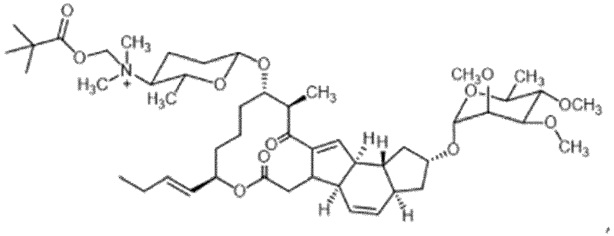 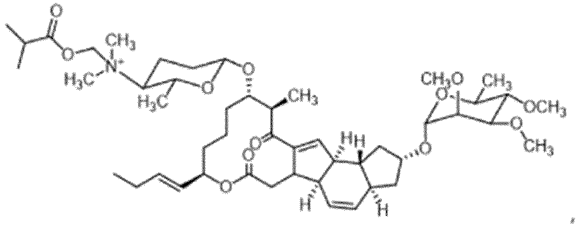 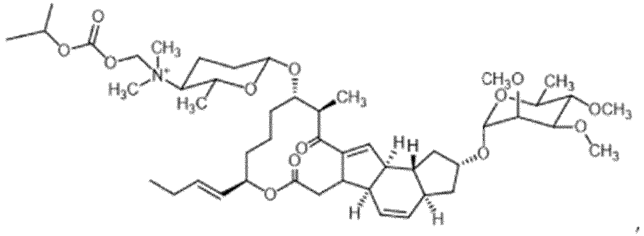 sous la forme d'un sel correspondant de celui-ci.

11. Composé selon l'une quelconque des revendications 1 à 10, dans lequel ledit sel correspondant est avec un anion choisi dans le groupe constitué par le chlorure, le sulfate, le nitrate, le phosphate, le citrate, le tartrate, l'acétate, le lactate, le propionate et le gluconate.

12. Composition comprenant un composé selon l'une quelconque des revendications 1 à 11 et un ou plusieurs adjuvants physiologiquement acceptables.

13. Utilisation d'un composé selon l'une quelconque des revendications 1 à 11 ou d'une composition selon la revendication 12 comme pesticide.

14. Procédé non thérapeutique de lutte contre un ravageur, tel qu'un ravageur de plante, comprenant la mise en contact d'un ravageur, tel qu'un ravageur de plante, avec un composé selon l'une quelconque des revendications 1 à 11 ou une composition selon la revendication 12.

15. Procédé de protection d'une plante contre un ravageur végétal, comprenant l'étape consistant à : appliquer un composé selon l'une quelconque des revendications 1 à 11 ou une composition selon la revendication 12 à une plante en ayant besoin.
